# EUROPEAN PATENT APPLICATION

(11) **EP 4 130 031 A1**
(43) Date of publication of application: **08.02.2023**
(21) Application number: 21781001.9
(22) Date of filing: 31.03.2021
(51) Int. Cl.: C07K 14/81, C07K 16/40, C12N 9/12, A61K 39/395

(54) **APPLICATIONS OF PI4K INHIBITOR IN INTRACELLULAR PROTEIN MISFOLDING-RELATED DISEASES AND LYSOSOMAL STORAGE DISEASES**

(30) Priority: 31.03.2020 CN 202010246586
(71) Applicant: Nuo-Beta Pharmaceutical Technology (Shanghai) Co., Ltd., Shanghai 201210 (CN)
(72) Inventor: HUANG, Fude, Shanghai 201210 (CN); WANG, Wenan, Shanghai 201210 (CN); HONG, Feng, Shanghai 201210 (CN); ZHENG, Linan, Shanghai 201210 (CN); JIAO, Changping, Shanghai 201210 (CN); CAO, Luxiang, Shanghai 201210 (CN); XIE, Yuyu, Shanghai 201210 (CN)
(74) Representative: Engelhard, Markus
(86) International application number: PCT/CN2021/084612
(87) International publication number: WO 2021/197389

(57) **Abstract**

Provided are uses of a PI4KIIIα specific inhibitor in preventing or treating intracellular protein misfolding-related diseases, also provided are uses of the PI4KIIIα specific inhibitor in preventing or treating lysosomal storage diseases.

## Description

### Technical Field

The present invention relates to use of a PI4KIIIα specific inhibitor in preventing or treating intracellular protein misfolding-related diseases, and meanwhile, the present invention relates to use of the PI4KIIIα specific inhibitor in preventing or treating lysosomal storage diseases.

### Background Art

Lysosomes are monolayer membrane-enclosed intracellular organs with vesicular structures, which contain many hydrolases capable of breaking down various foreign and endogenous macromolecules, including hydrolases capable of breaking down proteins, carbohydrates, lipids and the like, so that lysosomes are capable of breaking down biological macromolecules such as proteins, nucleic acids and polysaccharides, and also can digest the local cytoplasm or organelles of the cell itself. Lysosomes are classified into primary lysosomes and secondary lysosomes.

The primary lysosomes have a diameter of about 0.2 to 0.5 um, a membrane thickness of 7.5 nm, uniform content and no obvious particles, and are formed by secretion from Golgi apparatus. The primary lysosomes are formed in a budding form on the trans Golgi by the following processes: ribosome on endoplasmic reticulum synthesizes lysosomal protein, the lysosomal protein enters the endoplasmic reticulum cavity for N-linked glycosylation modification, and the lysosomal enzyme protein is firstly provided with 3 glucose, 9 mannose and 2 N-acetylglucosamine, then three molecules of glucose and one molecule of mannose are removed, and the resulting lysosomal enzyme protein enters Cis Golgi; N-acetylglucosamine phosphotransferase recognizes a signal plaque of lysosomal hydrolase; transfers N-acetylglucosamine phosphate onto 1 to 2 mannose residues; N-acetylglucosamine is cuts off by N-acetylglucosaminidase in medial Golgi to form an M6P ligand; the M6P ligand binds to receptors on cis Golgi; and the primary lysosomes are formed by selectively packaging. The primary lysosomes contain many hydrolases without activity, and the hydrolases have activity only when the lysosomes are ruptured or other materials enter. The hydrolases of the primary lysosomes include protease, nuclease, lipase, phosphatase, sulfatase and phosphatidase, more than 60 hydrolases are known, the hydrolases all belong to acid hydrolases, the optimum pH value for reaction is about 5, although the thickness of a lysosomal membrane is similar to that of a cytoplasmic membrane, the components of the lysosomal membrane are different from those of the cytoplasmic membrane, and the main differences are as follows: (1) the lysosomal membrane has a proton pump, and H+ is pumped into lysosomes to reduce the pH value of the lysosomes; and (2) the membrane proteins are highly glycosylated and may be beneficial in preventing degradation of the membrane proteins themselves.

The secondary lysosomes are all digestive vacuoles that are lysosomes undergoing or completing digestion, contain hydrolases and corresponding substrates, and can be classified into phagolysosome in which the material to be digested is derived from an external source and autophago lysosome in which the material to be digested is derived from various components of the cell itself.

The secondary lysosome is classified according to the source of the lysosomal substrates as:
(1) heterolysosome, which refers to macromolecular solutions or viruses, bacteria and the like that are impermeable to the cytoplasmic membrane, and the pinocytotic vesicles (or endosomes) formed by the former through pinocytosis (including receptor-mediated endocytosis) and the phagocytic vacuoles formed by the latter through phagocytosis, fuse with a primary lysosome (or endolysosome), respectively, to form a secondary lysosome (or lysosome);
(2) autolysosome or autophago lysosome, which refers to a secondary lysosome formed after fusion of autophagosome surrounding a partially damaged or aged organelle (mitochondria, endoplasmic reticulum fragments and the like) with a primary lysosome (or endolysosome). The material digested in the autolysosome or autophago lysosome is endogenous. The secondary lysosomes, which contain residual material that cannot be digested, are referred to as residual bodies. Some residual materials can be discharged, while some residual materials are stored in cells for a long time and are not discharged.

The residual bodies, also referred to as post-lysosomes, have lost enzymatic activity and only have undigested residues. The residual bodies can be discharged from the cells by efflux, and may also remain inside the cells and increase year by year, such as lipofuscin in hepatocytes.

Lysosomes are therefore a common junction downstream of multiple cellular metabolic pathways, such as autophagy, endocytosis and ER-Golgi pathway. Autophagy or autophago lysosome mediated signaling pathways are considered to be one of the major control systems for maintaining intracellular protein homeostasis (Manecka *et al*., 2017). The autophagy-lysosome signaling pathway is realized by two modes, namely macroautophagy and chaperon mediated autophagy (CMA). Macroautophagy begins mainly with the formation of autophagosomes, which fuse with lysosomes, introducing proteins into lysosomes for degradation (Bento *et al*., 2016). CMA is that Chaperons (e.g., Hsc70) bind to target proteins and traffic the proteins to lysosomes for degradation via the lysosomal receptor LAMP2A (Cuervo and Wong, 2014).

Lysosome function deficiency caused by the absence of hydrolases, insufficient activity of hydrolases, deficiency of activator protein, transporter protein or enzymes for lysosome protein processing and correction in lysosomes due to genetic variation and other factors causes the failure of digestion of corresponding substrates in secondary lysosomes, accumulation of substrates and metabolic disorder, thus leading to formation of lysosome storage disorders (LSDs). LSDs include more than 60 diseases including Gaucher's disease, Pompe disease and Niemann-Pick Type C disease (NPC). To date, main strategies for the treatment of such diseases are to block the synthesis of lysosomal enzyme substrate or replace lysosomal enzymes, but most treatments have limited efficacy (Platt *et al*., 2018). Therefore, clearance of the accumulated substances in lysosomes out of cells by promoting autophagy and lysosome secretion is a new therapeutic direction for LSDs (Samie and Xu, 2014).

Furthermore, in various protein inclusion body aggregation diseases (diseases caused by aggregation of intracellular proteins in inclusion bodies due to misfolding), expression and activity of various autophagy-lysosome regulators such as mTOR (Kahan, 2011, Decreassac *et al*., 2013, Magahaes *et al*., 2016, Tian *et al*., 2016), Beclin-1 (Spencer *et al*., 2009), Spermidin^{[14]} (Buttner *et al*., 2014), PLK2 (Mbefo *et al*., 2010) and LAMP2 (Xilouri *et al*., 2013) are varied to varying degrees. Proteins aggregated within these inclusion bodies, such as α-synuclein associated with Parkinson's disease (PD) and Lewy body dementia (LBD), are present in or degraded by the autophagy-lysosome pathway (Dehay et al., 2010. J. Neurosci, 30:12535-12544). Thus, promoting autophagy and lysosome secretion can also allow cells to promote clearance of these proteins, thereby reducing inclusion body aggregation.

The lysosomal contents contain many important enzymes, and the cellular wastes needed to be discharged outside the cell. Microglial lysosomes release their contents from cells by exocytosis, a part of secretion function of lysosomes is achieved by lysosomal exocytosis, and the exocytosis is an energy-dependent activity during which vesicles fuse with the cytoplasmic membrane and then release their contents into the extracellular space. Lysosomes can release their contents through complete fusion with the cytoplasmic membrane, or they can also undergo exocytosis by "kiss-and-run". With "kiss-and-run" exocytosis, the fusion of the lysosomal vesicles with the cytoplasmic membrane is transient and involves rapid opening and closing of the fusion pore to allow release of the contents of the vesicles.

Regulation of lysosome secretion (exocytosis) is expected to provide an effective therapeutic approach for the treatment of lysosomal storage diseases and cellular inclusion body aggregation diseases including Parkinson's disease and Lewy body dementia.

The level of autophagosome formation-associated genes GABARAPs/GATE-16 in brain tissue of patients with cellular inclusion body disease multiple system atrophy (MSA) is significantly reduced, indicating that autophagosome protein levels are inhibited in MSA patients and thus the maturation process of the autophagy system is disrupted (Tanji *et al*., 2013). Another research^{[22]} (Miki *et al*., 2016) showed that degradation of α-synuclein binding protein in MSA is regulated by the autophagy/beclin1 regulator 1 (AMBRA1) protein to which it is bound, and the level of AMBRA1 hyperphosphorylation in MSA neurons is significantly increased. *In vitro* experiments on mouse cortical cells showed that autophagy inhibitors can silence the activity of AMBRA1, resulting in aggregation of α-synuclein.

Folding and deposition of intracellular protein, such as tubulin at 15-18 nm in inclusion body myositis (IBM) is very common.

Some familial frontotemporal lobe degeneration may be associated with a mutation in progranulin (PGRN) gene. Progranulin is a growth factor, and gene mutation may cause reduction of the growth factor, which may hinder growth and repair of nerves. It has recently been found that abnormal phosphorylation of TAR-DNA binding protein 43 (TDP-43, a nuclear protein regulating transcription) is closely associated with frontotemporal lobe degeneration with motor neuron diseases.

Polyglutamine disease (PolyQ disease) consists of 9 diseases (Hungtington disease, (HD); X-associated spinal bulbar muscular atrophy (SBMA), dentatorubralpalludoluysian atrophy (DRPLA), and 6 spinal cerebellar atrophy (SCA 1, 2, 3, 6, 7 & 17)). Although the proteins associated with each disease are different, they are associated with the inactivation of the fluidity of autophagy^{[26]} (Cortes *et al*., 2015), and the autophagosome marker protein P62 can alleviate the deficits in behaviour and motor function in the SBMA mouse model^{[27]} (Doi *et al*., 2013). The DRPLA Drosophila model exhibits a phenotype similar to that of lysosomal storage diseases, with lipofuscin and P62 in the aggregates, indicating inactivation of lysosomal digestion function caused by impairment of autophagy ^{[28]} (Settembre *et al*., 2008).

In Hungtington disease, mutations in the Htt gene result in translation and accumulation of a length of abnormal (CAG-encoded) polyglutamines.

The motor neurons of 95% of amyotrophic lateral sclerosis (ALS) patients contain inclusion bodies mainly composed of TDP-43 protein. Other proteins such as FUS and SOD1 are also frequently co-aggregated together in ALS patients.

Prion disease is a disease caused by misfolded Prion protein (PrP) (Cai *et al*., 2016). Normal PrP (PrP^{C}) is present on the cell membrane, and it can also be converted to infectious form (PrP^{Sc}).

Phosphatidylinositol (PI) and its metabolic enzymes play an important role in lysosome formation and trafficking. It has been found that PI4KIIIβ can control the release of lysosomes and sorting of lysosomal materials ^{[38, 39]} (Sridhar S, Patel B, Aphkhazava D, et al. The lipid kinase PI4KIIIβ preserves lysosomal identity[J]. The EMBO Journal, 2012, 32(3):324-339; De Barry J, Janoshazi, Dupont J L, et al. Functional Implication of Neuronal Calcium Sensor-1 and Phosphoinositol 4-Kinase-beta Interaction in Regulated Exocytosis of PC12 Cells [J]. Journal of Biological Chemistry, 2006, 281(26):18098-18111). Both PI4KIIIβ and PI4KIIα in Golgi apparatus are involved in regulating or promoting delivery of lysosomal enzymes and proteins to lysosomes. Histologically, PI4KIIα gene-deleted mice show lipofuscin deposits, indicating that down-regulation of PI4KIIα inhibits endolysosome trafficking, leading to Purkinje cell reduction and axonal defects in the spinal cord. Inhibition of PI4KIIIβ and PI4KIIα leads to accumulation of lysosome proteins and Gaucher's disease (a lysosomal storage disorder) (Jovic M, Kean M J, Szentpetery Z, et al. Two phosphatidylinositol 4-kinases control lysosomal delivery of the Gaucher disease enzyme-glucocerebrosidase[J]. Molecular Biology of the Cell, 2012, 23(8):1533-1545). However, the role of PI4KIIIα in lysosome trafficking has not been reported.

PI4KIIIα, with scaffold proteins TTC7A or TTC7B and Efr3a or Efr3b located on the cell membrane, as well as cytoplasmic proteins FAM126A or FAM126B, or their homologous proteins, forms a protein complex on the cell membrane, and directly regulates the level of phosphatidylinositol-4 phosphate (PI4P) on the cell membrane; and the expression of PI4KIIIα or the location on the cell membrane depends on Efr3a/Efr3b, TTC7A/TTC7B and FAM126A/FAM126B (Baird D, Stefan C, et al., J Cell Biol, 2008, 183: 1061-1074; Nakatsu F, Baskin JM, et al., J Cell Biol, 2012, 199: 1003-1016; Baskin JM, Wu X et al., Nat Cell Biol, 2016, 18: 132-138; Zhang X, Jiang LX, et al., J Neurosci, 2017, 37(16): 4928-4941; Lees JA, Zhang Y, et al., PNAS, 2017, 114: 13720-13725).

### Summary of the Invention

In one aspect, the present invention provides use of a PI4KIIIα specific inhibitor in the preparation of a drug for preventing or treating intracellular protein misfolding-related diseases.

In another aspect, the present invention provides use of a PI4KIIIα specific inhibitor in the preparation of a drug for preventing or treating lysosomal storage diseases.

In some embodiments, the PI4KIIIα specific inhibitor is an antibody, a small molecule compound, an RNAi molecule or an antisense nucleic acid.

In some embodiments, the antibody is a monoclonal antibody or a polyclonal antibody.

In some embodiments, the antibody is a chimeric antibody, a humanized antibody or a fully human antibody.

In some embodiments, the RNAi molecule is a small interference RNA (siRNA), a short hairpin RNA (shRNA) or a microRNA (miRNA).

In some embodiments, the RNAi molecule is 18-100 bases in length.

In some embodiments, the RNAi molecule is modified to enhance its stability.

In some embodiments, the PI4KIIIα specific inhibitor is a small molecule compound.

In some embodiments, the small molecule compound is phenylarsine oxide or a derivative thereof, G1 and an analog thereof, A1 and an analog thereof.

In some embodiments, the phenylarsine oxide and the derivative thereof have a structure as represented by formula (I) or a pharmaceutically acceptable salt thereof, 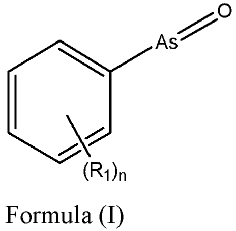 wherein each R₁ is independently selected from (a) H, halogen, nitro, cyano, hydroxyl, amino, carbamoyl, C₁₋₆ alkylsulfuryl, C₁₋₆ alkyl, C₁₋₆ cycloalkyl, C₂₋₆ alkynyl, C₂₋₆ alkenyl, C₁₋₆ alkoxy, C₁₋₆ haloalkyl, C₁₋₆ alkylene-NH₂, C₁₋₆ alkylene-NH-C(O)H, -As(O), -N=NH, N-(C₁₋₆ alkyl)amino, *N*,*N*-(C₁₋₆ alkyl)₂ amino, -NH-C(O)H, -NH-S(O)₂H, -C(O)OH, -OC(O)H, -SH,-S(O)₂H, -S(O)₂-NH₂ or heterocyclyl, and optionally substituted with R₂ or R₃, wherein the R₂ and the R₃ are each independently selected from amino, C₁₋₆ alkyl, C₁₋₆ alkoxy, C₁₋₆ haloalkyl, *N*-(C₁₋₆ alkyl) amino, *N*-(6-12 membered aryl)amino, *N*,*N*-(C₁₋₆ alkyl)₂ amino, C₃₋₆ cycloalkyl, 6-12 membered aryl or 3-12 membered heterocyclyl, and optionally substituted with one or more halogen, nitro, cyano, hydroxyl, amino, carbamoyl, -NH-C(O)-Rs, -C(O)OR₄, 6-12 membered aryl, C₁₋₆ alkyl, C₂₋₆ alkynyl, C₂₋₆ alkenyl, C₁₋₆ alkoxy, C₁₋₆ haloalkyl, 3-6 membered heterocyclyl, C₃₋₆ cycloalkyl or Bn-O-, R₄ is C₁₋₆ alkyl, and optionally substituted with one or more halogen, nitro, cyano, hydroxyl, amino, carbamoyl, 6-12 membered aryl, C₁₋₆ alkyl, C₂₋₆ alkynyl, C₂₋₆ alkenyl, C₁₋₆ alkoxy, C₁₋₆ haloalkyl, 3-6 membered heterocyclyl, C₃₋₆ cycloalkyl or Bn-O-, and R₅ is selected from H, C₁₋₆ alkyl, C₂₋₆ alkynyl, C₂₋₆ alkenyl, C₁₋₆ alkoxy or C₁₋₆ haloalkyl, and/or
(b) R₁ on two adjacent carbon atoms forms 5-12 membered cycloalkyl, aryl or heterocyclyl, and is optionally substituted with one or more halogen, nitro, cyano, hydroxyl, amino, carbamoyl, 6-12 membered aryl, C₁₋₆ alkyl, C₂₋₆ alkynyl, C₂₋₆ alkenyl, C₁₋₆ alkoxy, C₁₋₆ haloalkyl, 3-6 membered heterocyclyl, C₃₋₆ cycloalkyl or Bn-O-,
wherein n is an integer from 0 to 5.

In some embodiments, n is an integer from 0 to 2, and each of the R₁ is independently selected from H, halogen, nitro, cyano, hydroxyl, amino, carbamoyl, C₁₋₆ alkylsulfuryl, C₁₋₆ alkyl, C₁₋₆ cycloalkyl, C₁₋₆ alkoxy, C₁₋₆ haloalkyl, -As(O), *N*-(C₁₋₆ alkyl)amino, *N*,*N*-(C₁₋₆ alkyl)₂ amino, -NH-C(O)H or -NH-S(O)₂H, and optionally substituted with the R₂ or the R₃.

In some embodiments, n is an integer from 0 to 2, and each of the R₁ is independently selected from H, halogen, nitro, cyano, hydroxyl, amino, C₁₋₆ alkylsulfuryl, C₁₋₆ alkyl, C₁₋₆ cycloalkyl, C₁₋₆ alkoxy, C₁₋₆ haloalkyl, -As(O), -NH-C(O)H or -NH-S(O)₂H, and optionally substituted with the R₂ or the R₃.

In some embodiments, n is 1 or 2, each of the R₁ is independently selected from H, halogen, amino, C₁₋₆ alkylsulfuryl, C₁₋₆ cycloalkyl, C₁₋₆ alkoxy, C₁₋₆ haloalkyl, -NH-C(O)R₂ or -NH-S(O)₂R₃, wherein the R₂ is C₁₋₆ alkyl and optionally substituted with one 6-12 membered aryl, and the R₃ is 6-12 membered aryl and optionally substituted with one halogen, C₁₋₆ alkoxy or C₁₋₆ haloalkyl.

In some embodiments, the R₁ is located at ortho- and/or para-position of -As(O).

In some embodiments, n is 0.

In some embodiments, the small molecule compound is selected from the group consisting of the following compounds:

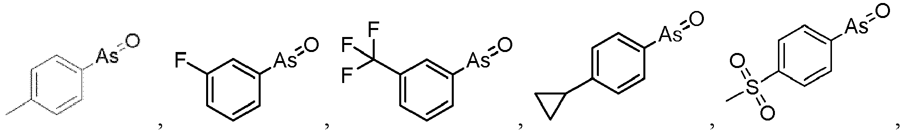

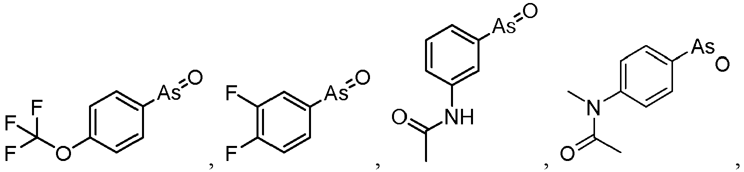

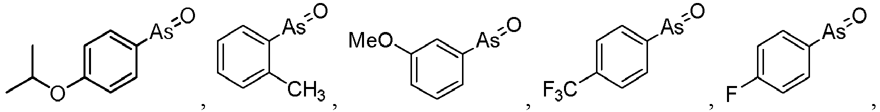

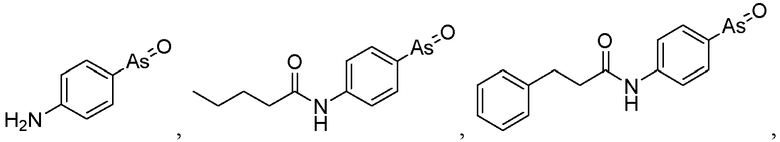

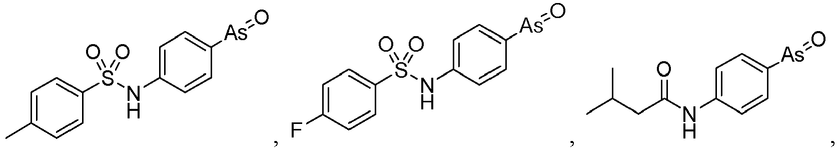

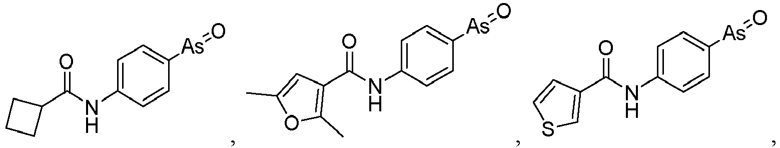

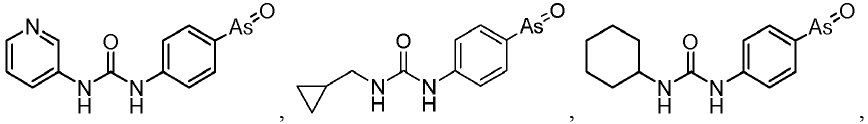

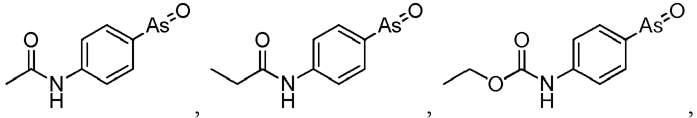

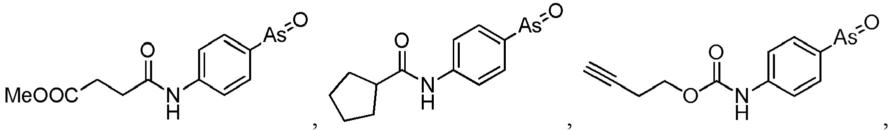

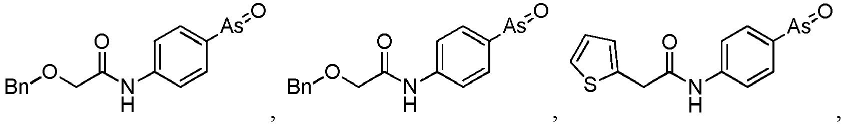

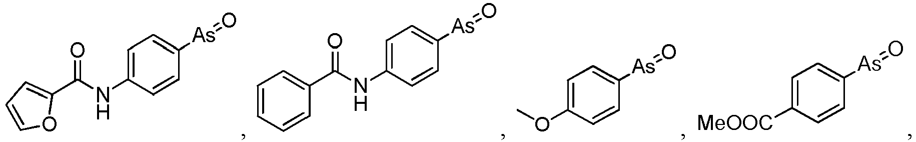

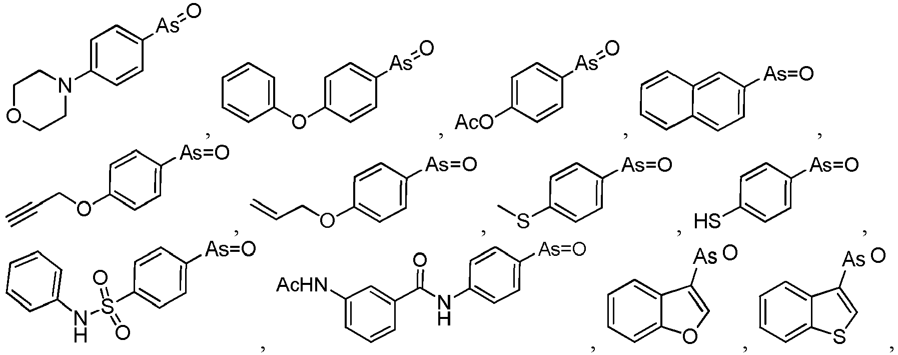

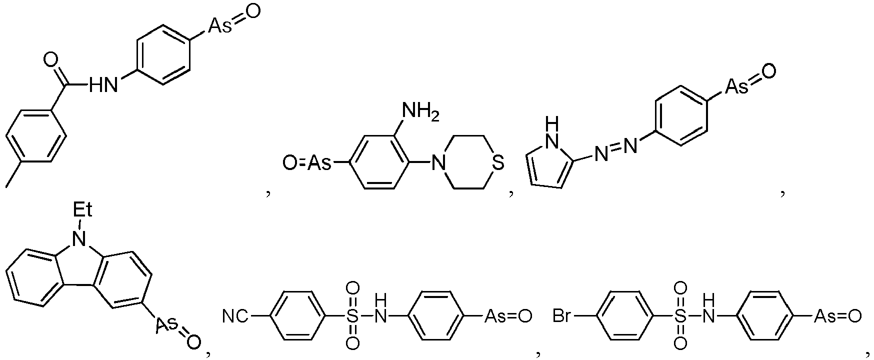

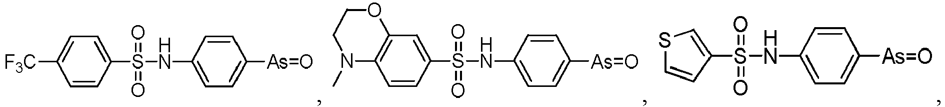

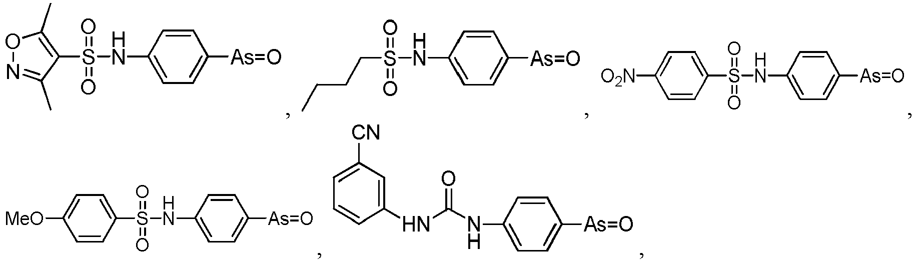

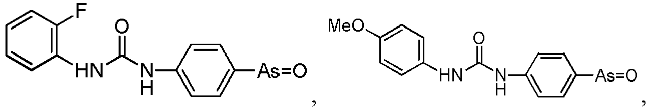

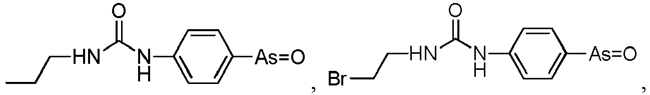

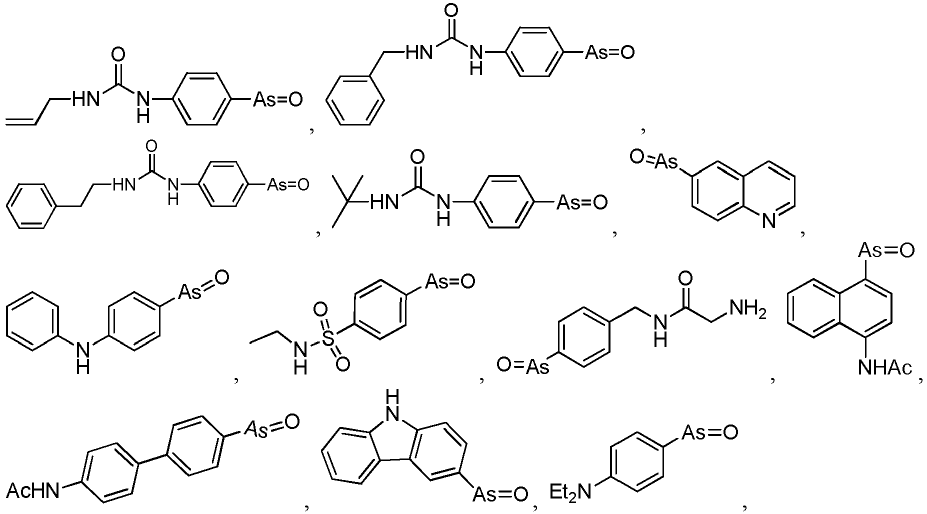

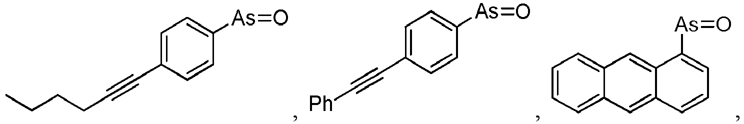

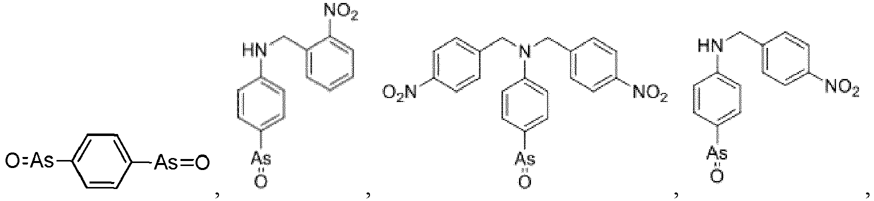

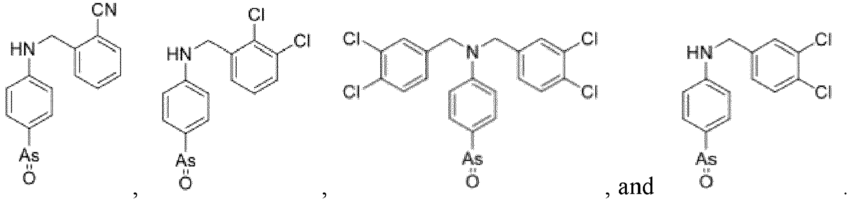

In some embodiments, the subject is a human or a mammal.

In some embodiments, the intracellular protein misfolding-related disease is Parkinson's disease, Lewy body dementia, multiple system atrophy, inclusion body myositis, frontotemporal dementia, Hungtington disease, polyglutamine disease, amyotrophic lateral sclerosis, or Prion disease.

In some embodiments, the lysosomal storage disease is sphingolipid metabolism disorder, mucopolysaccharidosis, glycogen storage disease, glycoprotein storage disease, lipid storage disease, post-translational modification deficiency, integral membrane protein deficiency disorder, neuronal ceroid lipofuscinosis, or lysosome-related organelle disorder.

In some embodiments, the sphingolipid metabolism disorder is Gaucher's disease.

In some embodiments, administering a second agent to a subject in need thereof is further comprised.

In some embodiments, the second agent is an agent for treating intracellular protein misfolding-related diseases or an agent for treating lysosomal storage diseases.

In some embodiments, the PI4KIIIα specific inhibitor is administered prior to, subsequent to or concurrently with the second agent.

In another aspect, the present invention provides a method for screening a drug for preventing or treating intracellular protein misfolding-related diseases, comprising contacting a drug candidate with a PI4KIIIα protein or nucleic acid or PI4KIIIα, and detecting whether the drug candidate is capable of inhibiting the formation or activity of PI4KIIIα.

In another aspect, the present invention provides a method for screening a drug for preventing or treating lysosomal storage diseases, comprising contacting a drug candidate with a PI4KIIIα protein or nucleic acid or PI4KIIIα, and detecting whether the drug candidate is capable of inhibiting the formation or activity of PI4KIIIα.

The present invention found that, contrary to the results produced by the deletion of PI4KIIα or PI4KIIIβ gene, inhibition of PI4KIIIα promotes lysosome secretion, reducing the accumulation or storage of various molecules in cells and lysosomes. The PI4KIIIα inhibitor such as a small molecule compound PAO can penetrate through the blood brain barrier, promote lysosome secretion, reduce accumulation or storage of various molecules in cells and lysosomes by inhibiting PI4KIIIα activity, thus making up for the defects of the existing therapy.

### Brief Description of the Drawings

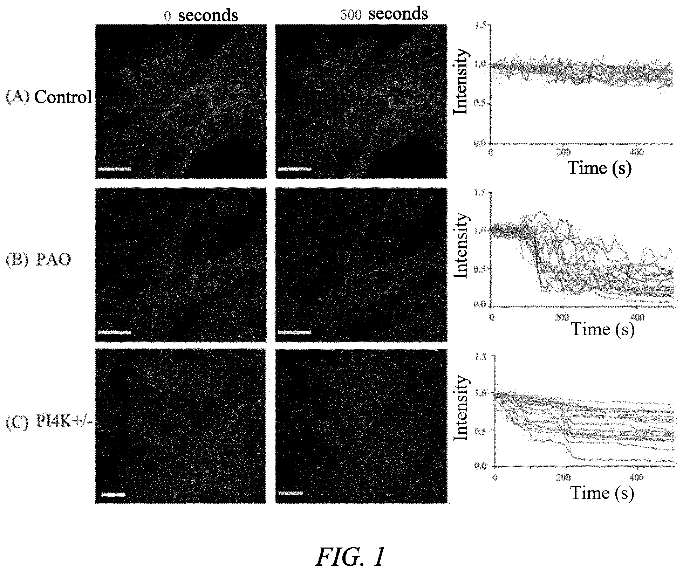

FIG. 1 shows that inhibition of PI4KIIIα induces microglial lysosomal exocytosis. (A) a control group where incubation is performed in loading solution, 27 spots are randomly selected, and the scale bar in FIG. 1(A) is 22 µm; (B) a 100 nM PAO treated group where 21 spots are randomly taken, and the scale bar in FIG. 1(B) is 22 µm; and (C) a PI4KIIIα heterozygous knockout group where 22 fluorescent spots are randomly taken, and the scale bar in FIG. 1(C) is 16 µm.

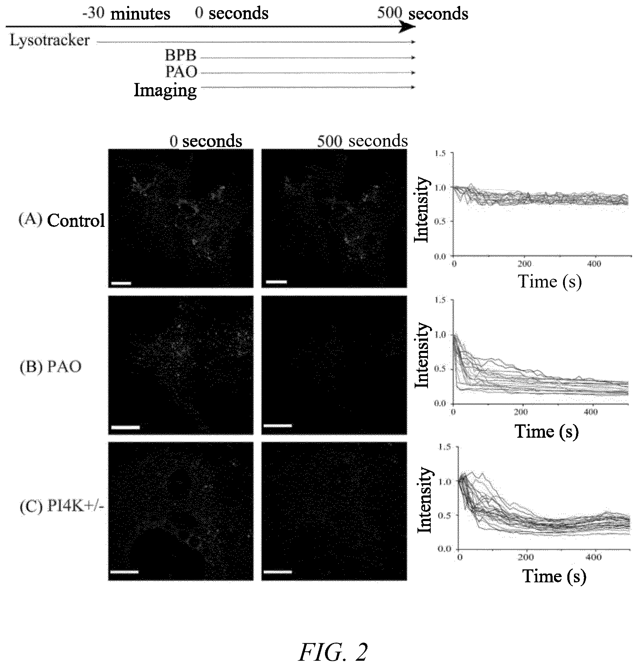

FIG. 2 shows that bromophenol blue (BPB) causes a decrease in lysosomal fluorescence by entering lysosomes of microglia due to "kiss-and-run" exocytosis. The pictures are fluorescence graphs for 0 and 500 seconds, respectively, and the line graphs are graphs for change of lysosomal fluorescence intensity. (A) a control group where WT cells are incubated in BPB solution at 0 seconds, 20 spots are randomly selected, and the scale bar in FIG. 2(A) is 16 µm; (B) a PAO treated group where WT cells are incubated in BPB and PAO solution at 0 second, 22 spots are randomly selected, and the scale bar in FIG. 2(B) is 22 µm; (C) a PI4KIIIα heterozygous knockout cell group where incubation is performed in BPB solution at 0 seconds, 22 spots are randomly taken, and the scale bar in FIG. 2(C) is 22 µm.

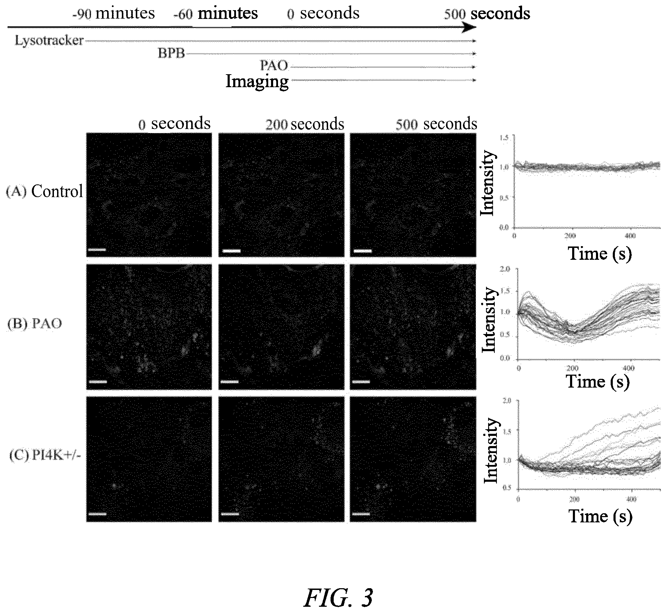

FIG. 3 shows recovery/increase of microglial lysosomal fluorescence following BPB exit from the lysosomes. The pictures are fluorescence graphs for 0, 200 and 500 seconds, respectively, and the line graphs are graphs for change of lysosomal fluorescence intensity with BPB solution added 60 minutes prior to drug administration. (A) a control group where WT cells are incubated in loading solution at 0 seconds, and 22 spots are randomly selected; (B) a PAO treated group where WT cells are incubated in PAO solution at 0 seconds, and 28 spots are randomly selected; (C) a PI4KIIIα heterozygous knockout cell group where incubation is performed in BPB solution at 0 seconds, and 26 spots are randomly taken. The scale bar in FIG. 3 is 22 µm.

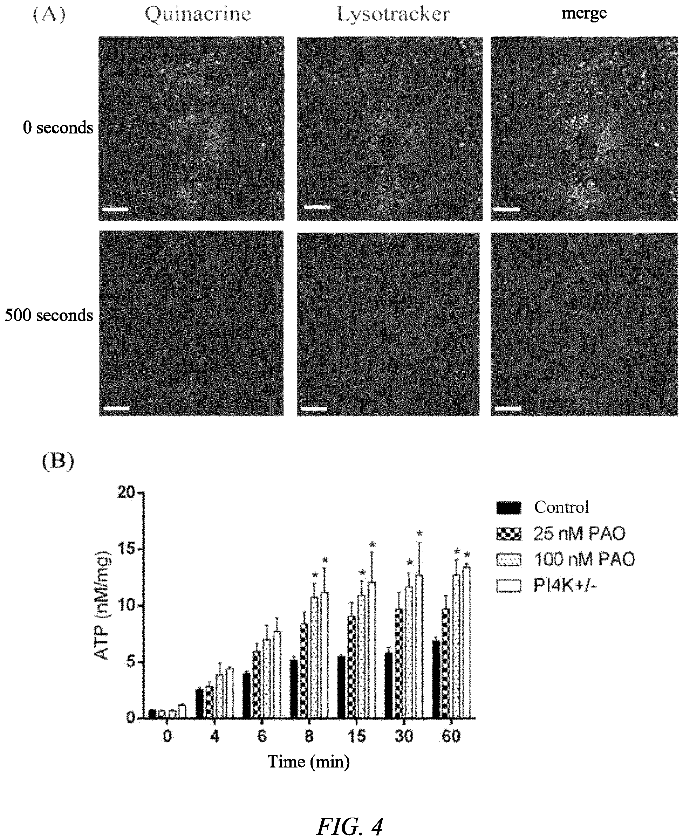

FIG. 4 shows that inhibition of PI4KIIIα enzyme activity promotes extracellular secretion of ATP by microglial lysosomes. (A) Co-localization of ATP with lysosomes. Left graph: 1 µM Quinacrine-labeled WT microglia intracellular ATP molecule, middle graph: LysoTracker-labeled lysosome, right graph: co-localized fluorescence graph after merging channels. The scale bar in FIG. 4 is 16 µm. (B) Extracellular ATP concentrations of 100 nM PAO-treated WT cell group and PI4K+/- cell group.

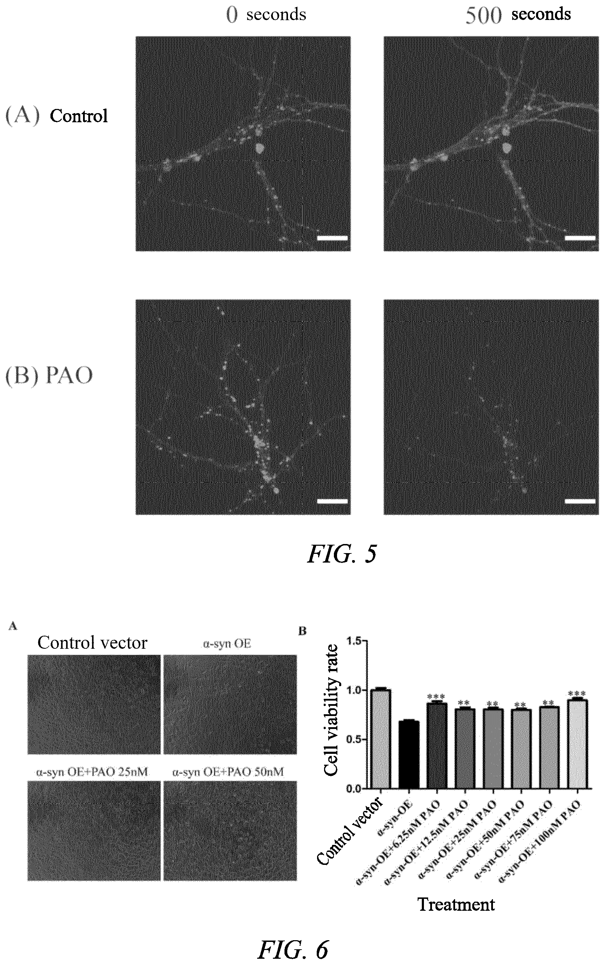

FIG. 5 shows inhibition of PI4KIIIα-induced lysosomal exocytosis of neuronal cells. Fluorescence pictures of lysosomes in hippocampal neurons are taken at 0 and 500 seconds. (A) A control group; (B) a 100 nM PAO treated group. The scale bar in FIG. 5 is 16 µm.
FIG. 6 shows that PAO inhibits cell death or apoptosis caused by over-expression of α-synuclein. PAO treatment is performed for 12 hours, MTT is added, the cells are incubated for 4 hours, and absorbance values are measured. (A) The pictures show cell survival; (B) Cell viability rate.

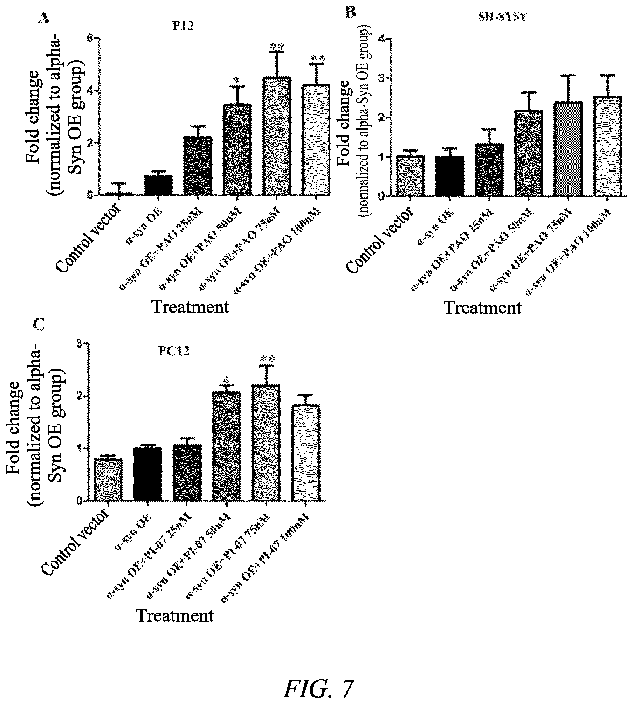

FIG. 7 shows that PAO and PI-07 promote α-synuclein secretion.

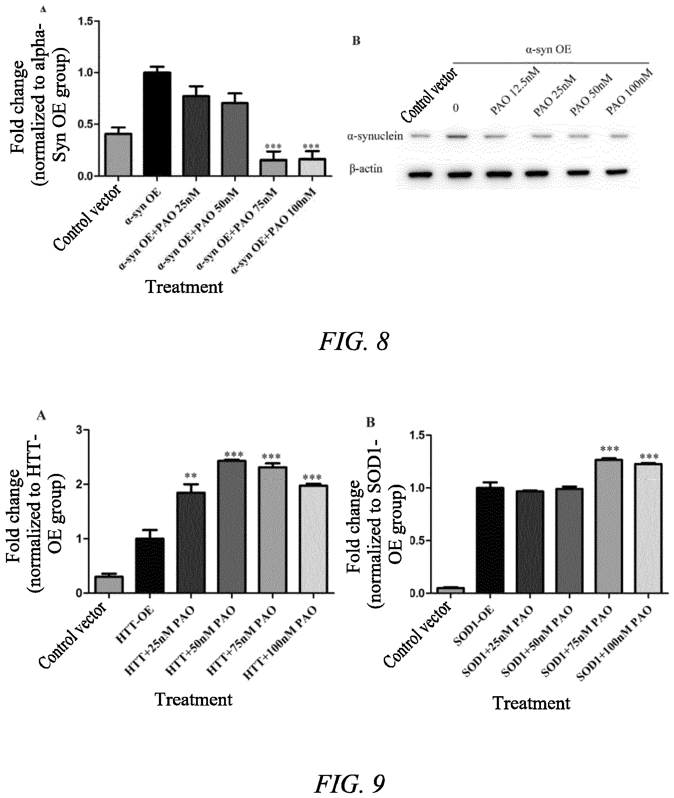

FIG. 8 shows that PAO down-regulates intracellular α-synuclein protein level. (A) ELISA detects intracellular α-synuclein protein level. n = 6, One-way ANOVA, **p < 0.001 vs. α-syn-OE. (B) Western blot detects intracellular α-synuclein protein level.
FIG. 9 shows that PAO promotes the secretion of HTT protein and SOD1 protein. (A) ELISA detects HTT protein secretion; (B) ELISA detects SOD1 protein.

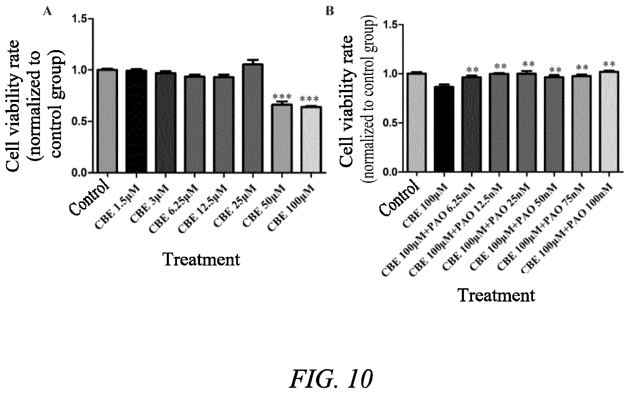

FIG. 10 shows the effect of PAO in reducing CBE-induced SH-SY5Y cell damage. MTT assay detects cell viability rate. (A) Treating SH-SY5Y cells by CBE with different concentrations; (B) treating for 24 hours with different concentrations of PAO after treating with 100 µM CBE. n = 5, One-way ANOVA, **p < 0.001, ***p < 0.0001, vs. α-syn-OE.

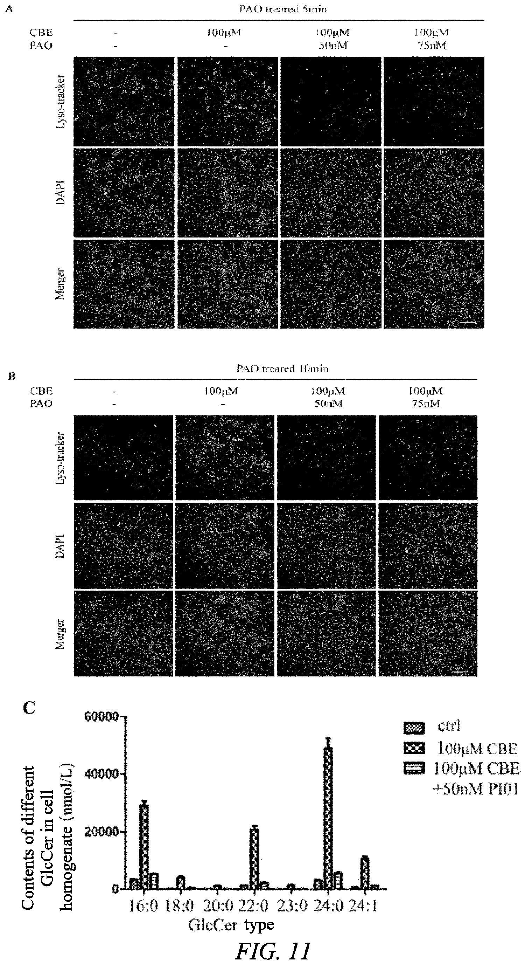

FIG. 11 shows that PAO alleviates CBE-induced lysosome aggregation and accumulation of glucosylceramide (GlcCer) within cells. (A) SH-SY5Y cells and lysosome tracker are incubated for 30 minutes, then the supernatant was discarded and replaced with PAO with different concentrations and incubated for 10 minutes, and the Lyso-tracker is observed by immunofluorescence; (B) the Lyso-tracker fluorescence intensity of each group is statistically analyzed; scale bar: 50 µm; n = 5; one-way ANOVA analysis, ##p < 0.001 compared to control group, **p < 0.001 and ^{∗∗∗}p < 0.0001 compared to 100 µM CBE treated group; (C) the GlcCer concentration in each group of cell lysates is statistically analyzed by LC/MS assays.

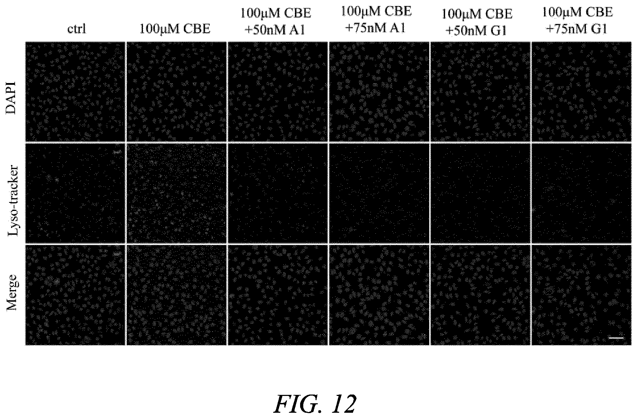

FIG. 12 shows that PI4KIIIa inhibitors A1 and G1 alleviate CBE-induced lysosome aggregation. SH-SY5Y was treated with 100 µM CBE for 72 hours, then Lysosome tracker (Lyso-tracker) was added to label lysosomes; after 30 minutes, 50 nM or 75 nM A1 or G1 was given for treatment for 5 minutes and 10 minutes, and observation was performed after 4% paraformaldehyde (PFA) fixation. Scale bar: 50 µm.

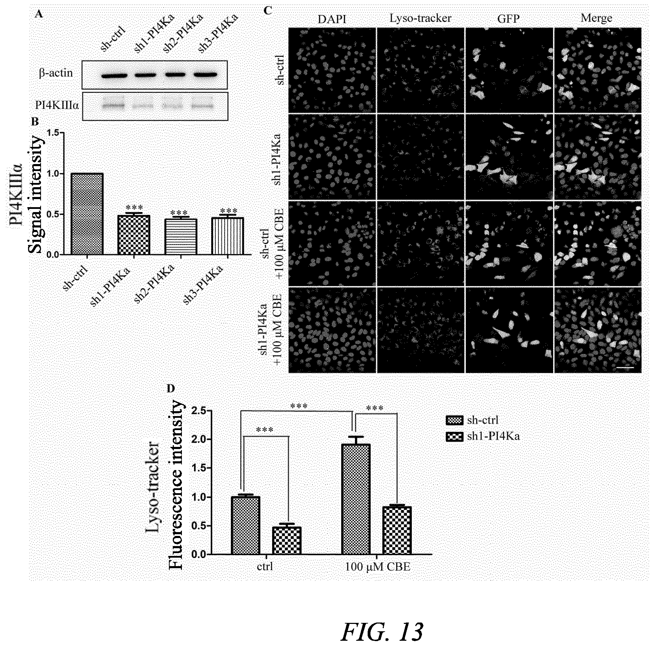

FIG. 13 shows that knockdown of *PI4Ka* promotes lysosomal exocytosis or reduces lysosomal storage. FIG. 13A shows that knockdown Western blot assay detects PI4KIIIα protein levels in SH-SY5Y cells treated with different shRNA lentiviral vectors (sh-ctrl,sh1-*PI4Ka*, sh2-*PI4Ka*, sh3-*PI4Ka*) for 48 hours; FIG. 13B is a statistical analysis of the Western blot assay results; and FIG. 13C shows the detection of Lyso-tracker fluorescence intensity after shRNA interference lentiviral vector treatment by immunofluorescence and statistical analysis (FIG. 13D). scale bar: 50 µm; n = 5; Data are expressed as mean ± SEM, One-way ANOVA analysis, ***p < 0.0001.

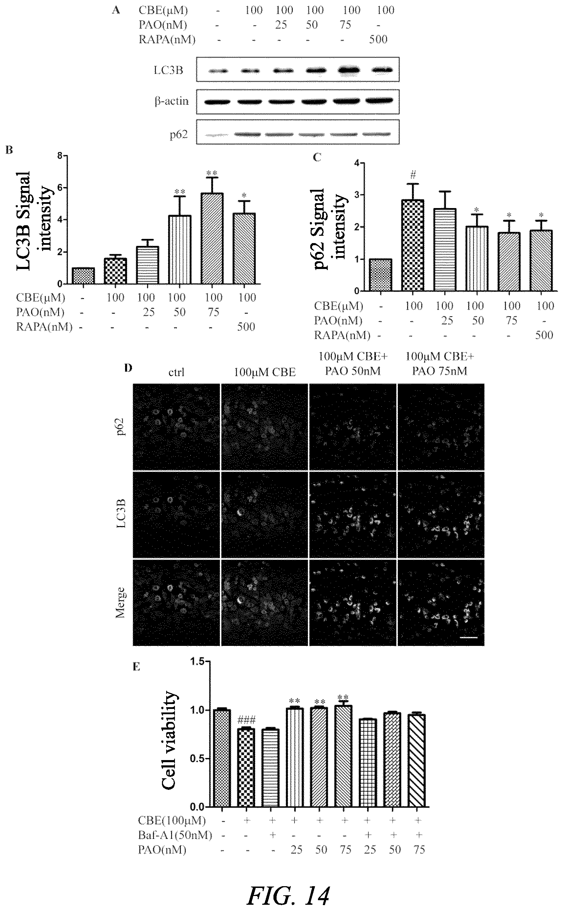

FIG. 14 shows PAO promotes the expression of LC3B and p62, Baf-A1 blocks PAO protection in cell models, where FIG. 34A shows the detection of LC3B and p62 proteins by Western blot assay; FIGs. 34B and 34C show statistical analysis of LC3B and p62 protein signal intensity by Image J software; FIG. 34D shows the detection of LC3B and p62 by immunofluorescence, red: p62, green: LC3B, scale bar: 50 µm; FIG. 34E shows that MTT determines cell viability for each group, and statistical analysis, n = 5, data are expressed as mean ± SEM, One-way ANOVA analysis, ###p < 0.0001 compared to control group, **p < 0.001 compared to 100 µM CBE treated group.

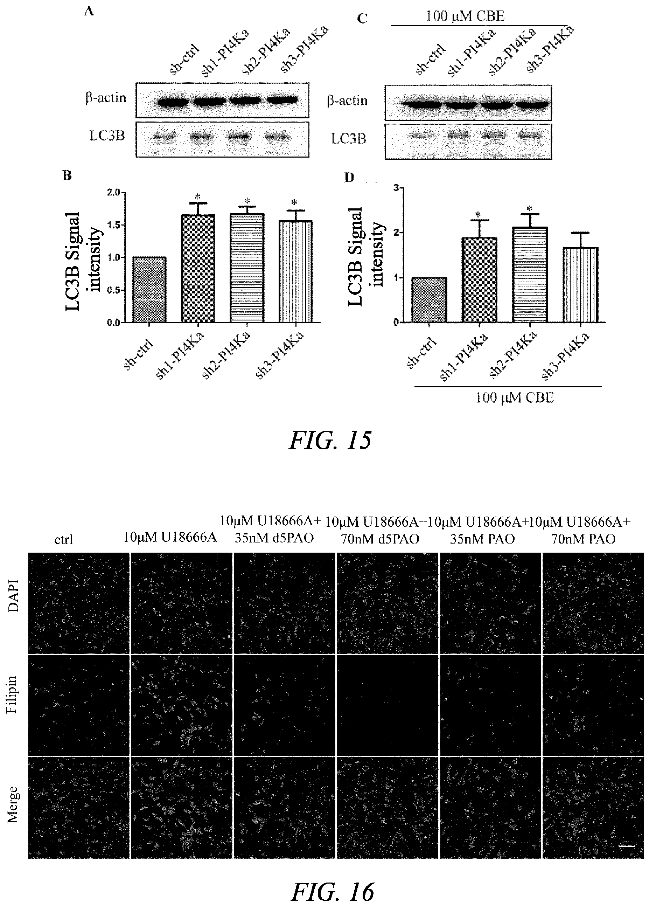

FIG. 15 shows the knockdown of *PI4Ka* activates the autophagy pathway in CBE-treated and CBE-free treated cells. PAO promotes the expression of LC3B and p62, and Baf-A1 blocks PAO protection in cell models. (A) Western blot assay detects LC3B and p62 protein; (B, C) statistical analysis of LC3B and p62 protein signal intensity by Image J software; (D) detection of LC3B and p62 by immunofluorescence, red: p62, green: LC3B, scale bar: 50 µm; (E) determination of cell viability for each group by MTT, and statistical analysis, n = 5, data are expressed as mean ± SEM, One-way ANOVA analysis, ###p < 0.0001 compared to control group, **p < 0.001 compared to 100 µM CBE treated group.
FIG. 16 shows inhibition by PAO of cholesterol storage caused by U18666A. Scale bar: 50 µm.

### Detailed Description of The Invention

Hereinafter, the present invention will be described in detail according to the embodiments and in conjunction with the accompanying drawings. The above aspects of the present invention and other aspects of the present invention will be apparent from the following detailed description. The scope of the present invention is not limited to the following embodiments.

### PI4KIIIα specific inhibitor

The term "PI4KIIIα specific inhibitor" as used herein refers to various substances capable of specifically reducing, decreasing or eliminating the transcription or translation of PI4KIIIα gene, and/or the activity of PI4KIIIα protein. In some embodiments, the PI4KIIIα specific inhibitor is capable of reducing the activity of PI4KIIIα by at least 5%, 10%, 20%, 40%, 50%, 80%, 90%, 95% or more. As used herein, "activity" when used in conjunction with an increase or decrease refers to a detected functional activity, which may be present in varying amounts, or in a constant amount but with a varying functional activity.

As used herein, the activity of PI4KIIIα refers to an activity of PI4KIIIα protein to phosphorylate phosphatidylinositol (PI) at a specific position (for example, to convert to phosphatidylinositol 4-phosphate (PI4P)). The binding constant of the PI4KIIIα specific inhibitor to PI4KIIIα protein is at least 2 times of the binding constant of the PI4KIIIα specific inhibitor to other non-specific binding proteins. In some embodiments, the PI4KIIIα specific inhibitor is capable of preferably recognizing PI4KIIIα protein in a complex mixture, including a mixture with other PI4K subtype proteins.

In some embodiments, the PI4KIIIα specific inhibitor inhibits PI4KIII at least 1 time, 2 times, 4 times, 5 times, 10 times, 20 times, 30 times, 50 times, 100 times, 200 times, 500 times, or 10,000 times more than other subtypes of PI4K protein (e.g., PI4K protein, including PI4KIIα or PI4KIIβ). For example, in some embodiments, the PI4KIIIα specific inhibitor does not substantially inhibit PI4KII (e.g., PI4KIIα or PI4KIIβ), e.g., IC₅₀ of the PI4KIIIα specific inhibitor is greater than or equal to 10 µM, 20 µM, 30 µM, 40 µM, 50 µM, 60 µM, 80 µM, 100 µM, 150 µM, 200 µM or 500 µM.

In some embodiments, the PI4KIIIα specific inhibitor inhibits PI4KIIIα at least 1 time, 2 times, 4 times, 5 times, 10 times, 20 times, 30 times, 50 times, 100 times, 200 times, 500 times, or 10,000 times stronger than other subtypes of PI4KIII (e.g., PI4KIIIβ).

In some embodiments, IC₅₀ of PI4KIIIα specific inhibitor against PI4KIIIα is less than or equal to 100 µM, 80 µM, 50 µM, 30 µM, 20 µM, 10 µM, 5 µM, 3 µM, 2 µM, 1 µM, 0.5 µM, 0.2 µM, 0.1 µM, 0.05 µM, 0.02 µM, 0.01 µM, 0.005 µM, 0.002 µM or 0.001 µM. In some embodiments, the PI4KIIIα specific inhibitor is an antibody, a small molecule compound, an RNAi molecule or an antisense nucleic acid.

In some embodiments, the PI4KIIIα specific inhibitor is an antibody.

The term "antibody" as used herein encompasses any immunoglobulin, monoclonal antibody, polyclonal antibody, multivalent antibody, bivalent antibody, monovalent antibody or antibody that binds a specific antigen. The term "antibody" herein is intended to encompass conventional four-chain antibodies as well as less conventional antibodies without four chains (e.g., antibodies naturally devoid of light chains).

One conventional intact antibody is a heterotetramer comprising two heavy (H) chains and two light (L) chains. Heavy chains of mammals may be classified into α chain, δ chain, ε chain, γ chain and µ chain, and each heavy chain consists of a variable region (VH) and a first constant region, a second constant region and a third constant region (CH1, CH2 and CH3, respectively); and light chains of mammals may be classified into λ chain or κ chain, and each light chain consists of a variable region (VL) and a constant region. A conventional antibody is "Y"-shaped, with the neck of the "Y"-shaped structure consisting of a second constant region and a third constant region of two heavy chains bound by a disulfide bond. Each arm of the "Y"-shaped structure comprises a variable region and a first constant region of one heavy chain, which binds to a variable region and a constant region of one light chain. The variable regions of the light and heavy chains determine antigen binding. The variable region of each chain contains three hypervariable regions, i.e., complementarity determining regions (CDRs) (the CDRs for the light chain include LCDR1, LCDR2 and LCDR3, and the CDRs for the heavy chain include HCDR1, HCDR2 and HCDR3). The three CDRs are interposed between flanking continuous portions known as framework regions (FRs), which are more highly conserved than the CDRs and form a scaffold to support the hypervariable loops. The constant regions of the heavy and light chains are not associated with antigen binding, but have multiple effector functions.

In some embodiments of the present invention, the antibody is a full-length antibody or an antigen-binding fragment.

The term "antigen-binding fragment" as used herein refers to an antibody fragment formed from the antibody fragment that contains an antibody portion with one or more CDRs but does not have an intact antibody structure. Examples of the antigen-binding fragment include, but are not limited to, an Fab fragment, an Fab' fragment, an F(ab')2 fragment, an Fv fragment, a single chain antibody molecules (scFv), an scFv dimer, a camelized single domain antibody and a nanobody. The antigen-binding fragment may bind to the same antigen as the parent antibody.

The "Fab" fragment of an antibody refers to the antibody portion that contains one light chain (including a variable region and a constant region) and a variable region and a first constant region of one heavy chain bound by a disulfide bond.

The "Fab'" fragment refers to an Fab fragment containing part of the hinge region.

The "F(ab')2" fragment refers to a dimer of Fab'.

The "Fv" fragment of an antibody consists of a variable region of one light chain and a variable region of one heavy chain.

The "single chain antibody molecule" or "scFv" refers to an engineered antibody composed of a light chain variable region and a heavy chain variable region directly connected or connected by a peptide chain. For detailed descriptions, see, e.g., Huston JS et al., Proc Natl AcadSci USA, 85:5879(1988).

The "scFv dimer" refers to a polymer formed from two scFvs.

The "camelized single domain antibody" (also referred to as "heavy chain antibody" or "heavy-chain-only antibodies (HCAb)") refers to an antibody that contains two heavy chain variable regions but no light chain. Heavy chain antibodies are originally derived from Camelidae (camels, dromedaries and llamas). Camelized antibodies have all the functions for antigen binding despite the deletion of light chains.

The "nanobody" consists of one heavy chain variable region and two constant regions CH2 and CH3 from a heavy chain antibody.

In some embodiments, the antibody is a monoclonal antibody or a polyclonal antibody.

In some embodiments, the antibody is a murine antibody, a rabbit antibody, a chimeric antibody, a humanized antibody or a fully human antibody.

The term "fully human" as used herein, when applied to an antibody or antigen-binding fragment, means that amino acid sequences of the antibody or antigen-binding fragment correspond to the amino acid sequence of an antibody produced by human or human immune cells or derived from a non-human source, e.g., a transgenic non-human animal utilizing a human antibody library, or other sequences encoding human antibodies.

The term "humanized" as used herein, when applied to an antibody or antigen-binding fragment, refers to an antibody or antigen-binding fragment that includes CDRs derived from a non-human animal, FR regions derived from a human, and constant regions derived from a human (when applicable). Because a humanized antibody or antigen-binding fragment has lower immunogenicity, it may be used as a therapeutic agent for human in certain embodiments. In certain embodiments, the non-human animals are mammals (e.g., mice, rats, rabbits, goats, sheep, guinea pigs or hamsters). In certain embodiments, the humanized antibody or antigen-binding fragment consists essentially entirely of human sequences, except for CDR sequences that are non-human.

The term "chimeric" as used herein, when applied to an antibody or antigen-binding fragment, refers to having a portion of the heavy and/or light chain derived from one species, with the rest of the heavy and/or light chain derived from an antibody or antigen-binding fragment from different species. In some embodiments, the chimeric antibody can include a constant region derived from a human and a variable region derived from a non-human animal (e.g., a mouse or a rabbit).

In some embodiments, the antibody described herein is a monospecific antibody, a bispecific antibody or a multispecific antibody.

In some embodiments, the antibody described herein may be further labeled.

In some embodiments, the PI4KIIIα specific inhibitor is an RNAi molecule.

The term "RNAi molecule" as used herein refers to RNA or an analog thereof, which has sufficient sequence complementarity with target RNA to guide RNA interference. In some embodiments, DNA is also included that may be used to generate RNA. RNA interference (RNAi) refers to a sequence-specific or selective process by which a target molecule (e.g., a target gene, a protein or RNA) is down-regulated.

In some embodiments, the RNAi molecule is capable of reducing expression of PI4KIIIα, e.g., knocking down PI4KA gene.

In some embodiments, the RNAi molecule has [5'- GCTGATCTCTACTACACTTCC - 3'] (SEQ ID NO: 1).

In some embodiments, the RNAi molecule has [5'- GGTTATCACCGGAAATCAATA -3'] (SEQ ID NO: 2).

In some embodiments, the RNAi molecule has [5'- GCAACATTATGCTGGACAAGA -3'] (SEQ ID NO: 3).

In some embodiments, the RNAi molecule is 18-100 bases in length.

In some embodiments, the RNAi molecule is modified to enhance its stability.

In some embodiments, the RNAi molecule is a small interference RNA (siRNA), a short hairpin RNA (shRNA) or a microRNA (miRNA).

The term "small interference RNA (siRNA)" as used herein refers to an RNA molecule, preferably a double-strand molecule, having a length of about 10 to 50 nucleotides, preferably a length of about 15 to 25 nucleotides, more preferably a length of about 17, 18, 19, 20, 21, 22, 23, 24 or 25 nucleotides, and the strand optionally has an overhanging terminus comprising, e.g., 1, 2 or 3 overhanging nucleotides (or nucleotide analogs). siRNA is capable of guiding or mediating degradation of RNA.

The term "short hairpin RNA (shRNA)" as used herein refers to an RNA molecule having a stem-loop structure that includes a first region and a second region of complementary sequences, complementarity degree and region direction are sufficient for base pairing to occur between the regions, the first and second regions are linked by a loop region, and the loop results from a lack of base pairing between nucleotides (or nucleotide analogs) in the loop region.

The term "microRNA or miRNA" as used herein is a short, naturally occurring, noncoding and single-stranded RNA molecule of about 16-26 nucleotides (nt) in length (e.g., about 16-29 nt, 19-22 nt, 20-25 nt or 21-23 nt), which is typically involved in regulating gene expression *in vivo.* In eukaryotic cells, the miRNA gene is transcribed by DNA transcriptase II into a "primary miRNA" (pri-miRNA) that is rapidly processed by ribonuclease III (Drosha) into a "precursor" miRNA (pre-miRNA), and the pre-miRNA is transported from cell nucleus into cytoplasm, and then recognized and cleaved into mature miRNA by another ribonuclease III (Dicer). Mature miRNA molecules are partially complementary to one or more mRNAs and regulate the expression of proteins. The sequences of known miRNAs may be obtained from published databases, such as the miRBase database (www.mirbase.org), where information is provided including miRNA sequence information, functional annotations, predicted gene targets and the like. In the present invention, miRNA further includes RNA molecules expressed in cells by synthetic plasmids, having a structure and function similar to natural miRNA, and capable of targeting the corresponding mRNA like natural miRNA and preventing its translation into protein.

In some embodiments, the PI4KIIIα specific inhibitor is an antisense nucleic acid.

The term "antisense nucleic acid" as used herein includes nucleotides that are fully complementary to a target sequence as well as those nucleotides that have one or more nucleotide mismatches, so long as the antisense nucleic acid is capable of specifically hybridizing to the target sequence. For example, the antisense nucleic acid herein includes a polynucleotide having at least 70% or more, preferably 80% or more, more preferably 90% or more, and even more preferably 95% or more homology over a length of at least 15 consecutive nucleotides. Due to the formation of a hybrid, the transcription of the target gene and/or translation of the target mRNA is reduced or blocked.

In some embodiments, the PI4KIIIα inhibitor is a small molecule compound.

The term "small molecule compound" as used herein refers to an organic compound having a molecular weight of less than 3000, 2500, 2000, 1500, 1000 or 500 Daltons, which may be natural or chemically synthesized.

In some embodiments, the phenylarsine oxide and the derivative thereof have a structure as represented by formula (I) or a pharmaceutically acceptable salt thereof, 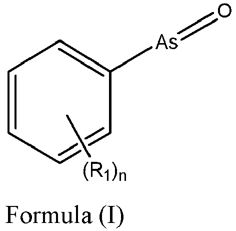 wherein each R₁ is independently selected from (a) H, halogen, nitro, cyano, hydroxyl, amino, carbamoyl, C₁₋₆ alkylsulfuryl, C₁₋₆ alkyl, C₁₋₆ cycloalkyl, C₂₋₆ alkynyl, C₂₋₆ alkenyl, C₁₋₆ alkoxy, C₁₋₆ haloalkyl, C₁₋₆ alkylene-NH₂, C₁₋₆ alkylene-NH-C(O)H, -As(O), -N=NH, N-(C₁₋₆ alkyl)amino, *N*,*N*-(C₁₋₆ alkyl)₂ amino, -NH-C(O)H, -NH-S(O)₂H, -C(O)OH, -OC(O)H, -SH,-S(O)₂H, -S(O)₂-NH₂ or heterocyclyl, and optionally substituted with R₂ or R₃, wherein the R₂ and the R₃ are each independently selected from amino, C₁₋₆ alkyl, C₁₋₆ alkoxy, C₁₋₆ haloalkyl, *N*-(C₁₋₆ alkyl) amino, *N*-(6-12 membered aryl)amino, *N*,*N*-(C₁₋₆ alkyl)₂ amino, C₃₋₆ cycloalkyl, 6-12 membered aryl or 3-12 membered heterocyclyl, and optionally substituted with one or more halogen, nitro, cyano, hydroxyl, amino, carbamoyl, -NH-C(O)-Rs, -C(O)OR₄, 6-12 membered aryl, C₁₋₆ alkyl, C₂₋₆ alkynyl, C₂₋₆ alkenyl, C₁₋₆ alkoxy, C₁₋₆ haloalkyl, 3-6 membered heterocyclyl, C₃₋₆ cycloalkyl or Bn-O-, R₄ is C₁₋₆ alkyl, and optionally substituted with one or more halogen, nitro, cyano, hydroxyl, amino, carbamoyl, 6-12 membered aryl, C₁₋₆ alkyl, C₂₋₆ alkynyl, C₂₋₆ alkenyl, C₁₋₆ alkoxy, C₁₋₆ haloalkyl, 3-6 membered heterocyclyl, C₃₋₆ cycloalkyl or Bn-O-, and R₅ is selected from H, C₁₋₆ alkyl, C₂₋₆ alkynyl, C₂₋₆ alkenyl, C₁₋₆ alkoxy or C₁₋₆ haloalkyl, and/or
(b) R₁ on two adjacent carbon atoms forms 5-12 membered cycloalkyl, aryl or heterocyclyl, and is optionally substituted with one or more halogen, nitro, cyano, hydroxyl, amino, carbamoyl, 6-12 membered aryl, C₁₋₆ alkyl, C₂₋₆ alkynyl, C₂₋₆ alkenyl, C₁₋₆ alkoxy, C₁₋₆ haloalkyl, 3-6 membered heterocyclyl, C₃₋₆ cycloalkyl or Bn-O-,
wherein n is an integer from 0 to 5.

In some embodiments, n is an integer from 0 to 2, and each of the R₁ is independently selected from H, halogen, nitro, cyano, hydroxyl, amino, carbamoyl, C₁₋₆ alkylsulfuryl, C₁₋₆ alkyl, C₁₋₆ cycloalkyl, C₁₋₆ alkoxy, C₁₋₆ haloalkyl, -As(O), *N*-(C₁₋₆ alkyl)amino, *N*,*N*-(C₁₋₆ alkyl)₂ amino, -NH-C(O)H or -NH-S(O)₂H, and optionally substituted with the R₂ or the R₃.

In some embodiments, n is an integer from 0 to 2, and each of the R₁ is independently selected from H, halogen, nitro, cyano, hydroxyl, amino, C₁₋₆ alkylsulfuryl, C₁₋₆ alkyl, C₁₋₆ cycloalkyl, C₁₋₆ alkoxy, C₁₋₆ haloalkyl, -As(O), -NH-C(O)H or -NH-S(O)₂H, and optionally substituted with the R₂ or the R₃.

In some embodiments, n is 1 or 2, each of the R₁ is independently selected from H, halogen, amino, C₁₋₆ alkylsulfuryl, C₁₋₆ cycloalkyl, C₁₋₆ alkoxy, C₁₋₆ haloalkyl, -NH-C(O)R₂ or -NH-S(O)₂R₃, wherein the R₂ is C₁₋₆ alkyl and optionally substituted with one 6-12 membered aryl, and the R₃ is 6-12 membered aryl and optionally substituted with one halogen, C₁₋₆ alkoxy or C₁₋₆ haloalkyl.

In some embodiments, the R₁ is located at ortho- and/or para-position of -As(O).

In some embodiments, n is 0.

The term "substituted" as used herein, when referring to a chemical group, means that one or more hydrogen atoms of the chemical group are removed and substituted with a substituent.

The term "substituent" as used herein has general meaning known in the art and refers to a chemical moiety covalently attached to, or fused, where appropriate, to a parent group.

The term "Cₙ-Cₘ" as used herein represents a range of numbers of carbon atoms, where n and m are integers and the range of numbers of carbon atoms includes the endpoints (i.e., n and m) and each integer point therebetween. For example, C₁-C₆ represents a range of 1 to 6 carbon atoms, including 1 carbon atom, 2 carbon atoms, 3 carbon atoms, 4 carbon atoms, 5 carbon atoms and 6 carbon atoms.

The term "alkyl" as used herein, whether used as a part of another term or used alone, refers to a saturated hydrocarbon group, which may be linear or branched. The term "Cₙ-Cₘ alkyl" refers to alkyl having n to m carbon atoms. In certain embodiments, the alkyl group contains 1 to 12, 1 to 8, 1 to 6, 1 to 4, 1 to 3, or 1 to 2 carbon atoms. Examples of the alkyl group include, but are not limited to, chemical groups such as methyl, ethyl, n-propyl, isopropyl, n-butyl, tert-butyl, isobutyl, sec-butyl, 2-methyl-1-butyl, n-pentyl, 3-pentyl, n-hexyl, 1,2,2-trimethylpropyl.

The term "alkenyl" as used herein, whether used as a part of another term or used alone, refers to an unsaturated hydrocarbyl group, which may be a linear or branched group having at least one carbon-carbon double bond. In certain embodiments, the alkenyl group contains 2 to 12, 2 to 10, 2 to 8, 2 to 6, 2 to 5, 2 to 4, or 2 to 3 carbon atoms. In certain embodiments, the alkenyl group can also have 1 to 6, 1 to 5, 1 to 4, 1 to 3, 1 to 2, or 1 carbon-carbon double bond. Examples of the alkenyl group include, but are not limited to, chemical groups such as ethenyl, n-propenyl, isopropenyl, n-butenyl, sec-butenyl.

The term "alkynyl" as used herein, whether used as a part of another term or used alone, refers to an unsaturated alkynyl group, either linear or branched, having at least one carbon-carbon triple bond. In certain embodiments, the alkynyl group contains 2 to 12, 2 to 10, 2 to 8, 2 to 6, 2 to 5, 2 to 4, or 2 to 3 carbon atoms. In certain embodiments, the alkynyl group can also have 1 to 6, 1 to 5, 1 to 4, 1 to 3, 1 to 2, or 1 carbon-carbon triple bond. Examples of the alkynyl group include, but are not limited to, chemical groups such as ethynyl, propynyl and butynyl.

The term "cycloalkyl" as used herein refers to cyclic alkyl consisting of at least 3 atoms. The term "n-m membered cycloalkyl" refers to cycloalkyl having n to m members forming a ring. Furthermore, the ring may also have one or more double bonds, but does not have a fully conjugated system. In certain embodiments, the cycloalkyl has 3 to 8, 3 to 6, or 4 to 6 carbon atoms forming a ring. Examples of the cycloalkyl include, but are not limited to, cyclopropane, cyclobutane, cyclopentyl and the like.

The term "heterocyclyl" as used herein, refers to a cyclic group in which at least one atom in the ring system is a heteroatom and the remaining ring atoms are carbon atoms. The term "n-m membered heterocyclyl" refers to heterocyclyl having n to m members forming a ring. The term "heterocyclyl" as used herein includes heteroaryl and heterocycloalkyl. In addition, the ring may also have one or more double bonds. In certain embodiments, the heterocyclyl is saturated heterocycloalkyl. Examples of the heteroatom include, but are not limited to, oxygen, sulfur, nitrogen, phosphorus and the like.

The term "heterocycloalkyl" as used herein refers to cycloalkyl in which at least one atom in the ring system is a heteroatom and the remaining ring atoms are carbon atoms. The term "n-m membered heterocycloalkyl" refers to heterocycloalkyl having n to m members forming a ring. Furthermore, the ring may also have one or more double bonds, but does not have a fully conjugated system. In certain embodiments, the heterocycloalkyl is saturated heterocycloalkyl. Examples of the heteroatom include, but are not limited to, oxygen, sulfur, nitrogen, phosphorus and the like. In certain embodiments, the heterocycloalkyl has 3 to 8, 3 to 6, or 4 to 6 carbon atoms forming a ring. Examples of the heterocycloalkyl include, but are not limited to, azetidine, aziridine, pyrrolidinyl, piperidinyl, piperazinyl, morpholinyl, thiomorpholine, homopiperazine and the like.

The term "aryl" as used herein, whether used as a part of another term or used alone, refers to a mono-or poly-carbocyclic ring system group having alternating double and single bonds between carbon atoms forming a ring. The term "Cₙ-Cₘ aryl" refers to aryl having n to m carbon atoms forming a ring. In certain embodiments, the aryl ring system has 6 to 12, 6 to 10, or 6 to 8 carbon atoms in one or more rings. In certain embodiments, the aryl ring system has 2 or more rings fused together. Examples of the aryl group include, but are not limited to, chemical groups such as phenyl, naphthyl, tetrahydronaphthyl, indanyl, indenyl and the like.

The term "heteroaryl" as used herein refers to an aryl group in which at least one ring atom in the aromatic ring is a heteroatom and the remaining ring atoms are carbon atoms. The term "n-m membered heteroaryl" refers to heteroaryl having n to m members forming a ring. Examples of the heteroatom include, but are not limited to, oxygen, sulfur, nitrogen, phosphorus and the like. In certain embodiments, the heteroaryl can have 5 to 10, 5 to 8, or 5 to 6 members forming a ring. In certain embodiments, the heteroaryl is 5- or 6-membered heteroaryl. Examples of the heteroaryl include, but are not limited to, furyl, thienyl, pyridyl, quinolyl, pyrrolyl, N lower alkylpyrrolyl, pyridyl-N-oxide, pyrimidinyl, pyrazinyl, imidazolyl, indolyl and the like.

The term "alkoxy" as used herein, whether used as a part of another term or used alone, refers to a group as represented by formula "-O-alkyl". The term "Cₙ-Cₘ alkoxy" means that the alkyl moiety of the alkoxy has n to m carbon atoms. In certain embodiments, the alkyl moiety has 1 to 6, 1 to 4, or 1 to 3 carbon atoms. Examples of the alkoxy group include, but are not limited to, chemical groups such as methoxy, ethoxy, propoxy (e.g., n-propoxy and isopropoxy), t-butoxy and the like.

The term "haloalkyl" as used herein, whether used as a part of another term or used alone, refers to a group as represented by formula "-alkyl-X", wherein X is a halogen, an atom selected from fluorine, chlorine, bromine and iodine. The term "Cₙ-Cₘ haloalkyl" means that the alkyl moiety of the haloalkyl has n to m carbon atoms. In certain embodiments, the alkyl moiety has 1 to 6, 1 to 4, or 1 to 3 carbon atoms. Examples of the haloalkyl group include, but are not limited to, chemical groups such as halomethyl, haloethyl, halopropyl (e.g., n-halopropyl and iso-halopropyl), t-halobutyl and the like.

The term "n-membered" as used herein, wherein n is an integer, is commonly used with a ring system to describe the number of atoms forming a ring in the ring system. For example, piperidinyl is one example of a 6-membered heterocycloalkyl ring, pyrazolyl is one example of a 5-membered heteroaryl ring, pyridinyl is one example of a 6-membered heteroaryl ring and 1,2,3,4-tetrahydro-naphthalene is one example of 10-membered aryl. The term "n- to m-membered" as used herein is generally used with a ring system to describe the number of members forming a ring in the ring system, where n and m are integers and the range of members forming a ring includes the endpoints (i.e., n and m) and each integer point therebetween. For example, 3- to 8-membered means a range of 3 to 8 members forming a ring, including 3 members, 4 members, 5 members, 6 members, 7 members and 8 members.

The term "halogen" as used herein refers to an atom selected from fluorine, chlorine, bromine and iodine.

The term "cyano" as used herein refers to a group as represented by formula "-CN".

The term "hydroxyl" as used herein refers to a group as represented by formula "-OH".

The term "nitro" as used herein refers to a group as represented by formula "-NOz".

The term "amino" as used herein refers to a group as represented by formula "-NH₂".

The term "carbamoyl" as used herein refers to a group as represented by formula "-HNCONH₂".

The term "compound" as used herein is intended to include all stereoisomers (e.g., enantiomers and diastereomers), geometric isomers, and tautomers of the structures shown.

The compounds described herein may be asymmetric (e.g., having one or more stereocenters). Unless otherwise indicated, all stereoisomers, such as enantiomers and diastereomers, are intended to be included. Various geometric isomers including olefins, carbon-carbon double bonds and the like may also be present in the compounds described herein, and all such stable isomers have been contemplated herein. *Cis-* and trans-geometric isomers of the compounds are described herein and may be isolated as mixtures of isomers or as individual isomers.

The compounds herein also include tautomeric forms of the compounds. Tautomeric forms result from the exchange of a single bond with an adjacent double bond accompanied by the migration of a proton. Tautomeric forms include tautomers of protons in isomeric protonated states with the same chemical formula and total charge. Examples of the proton tautomers include keto-enol pairs, amide-imidic acid pairs, lactam-lactim pairs, enamine-imine pairs and cyclic forms, in which a proton can occupy two or more positions of a heterocyclic system, such as 1H- and 3H-imidazole, *1H-, 2H-* and 4H-1,2,4-triazole, 1H- and 2*H*-isoindole and 1H- and 2H-pyrazole. Tautomeric forms can be in equilibrium or sterically locked into one form by appropriate substitution.

In certain embodiments, the small molecule compound herein can be obtained by organic synthesis. The compounds herein, including salts, esters, hydrates, or solvates thereof, may be prepared using any of the well known organic synthetic techniques and may be synthesized according to a variety of possible synthetic routes.

In some embodiments, the small molecule compound described herein is a compound having the following structural formula, including one or more of:

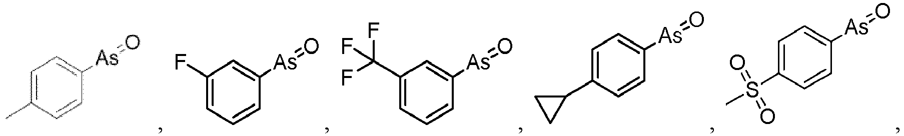

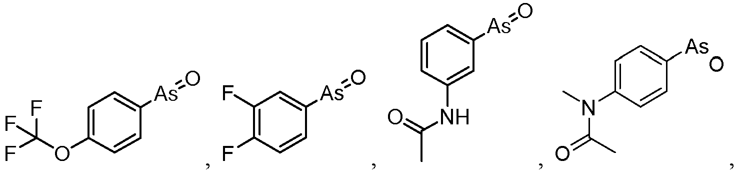

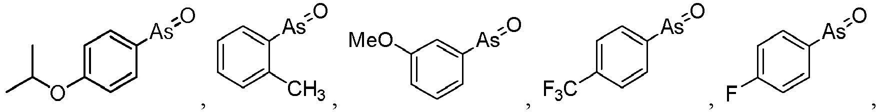

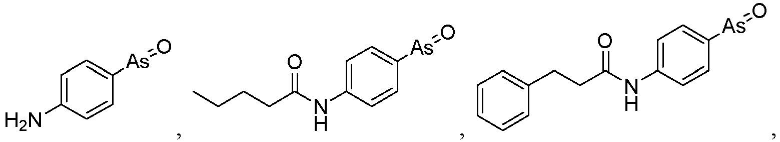

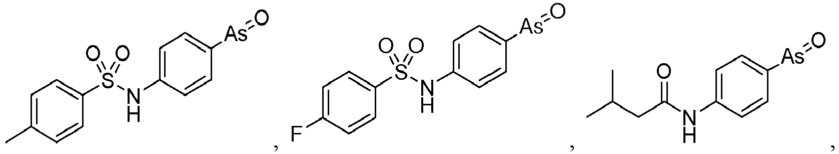

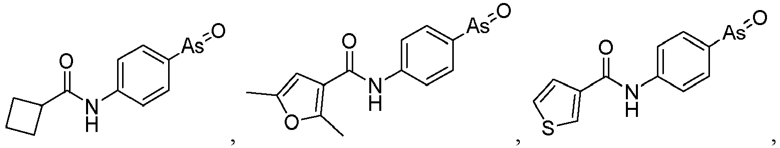

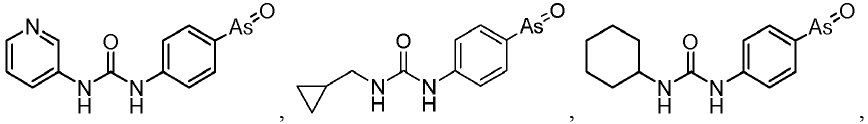

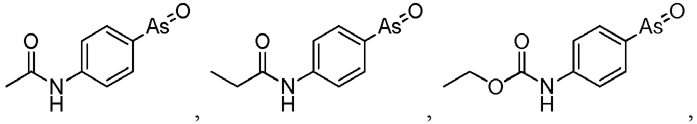

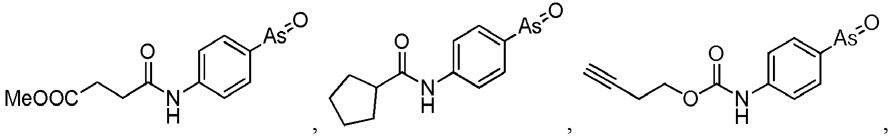

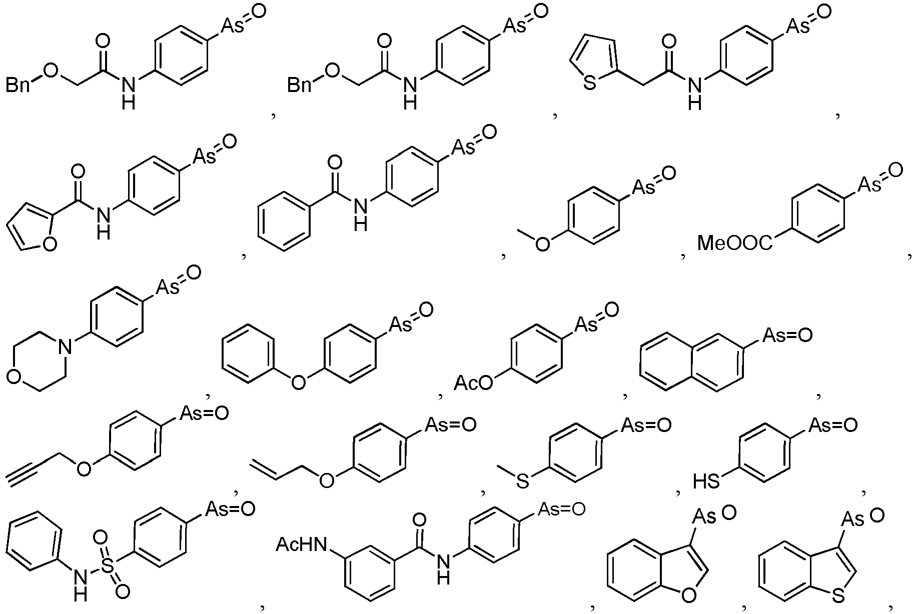

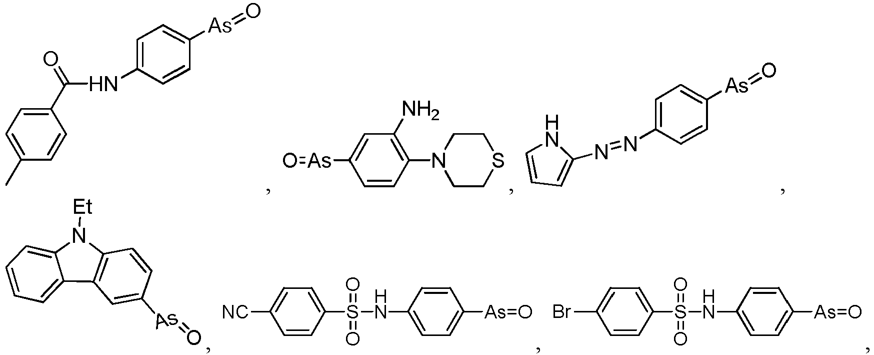

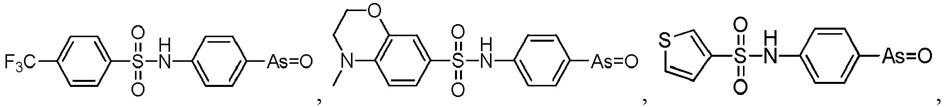

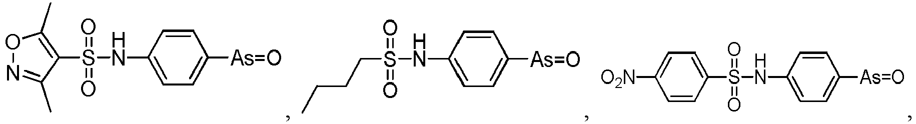

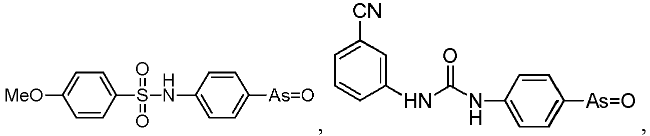

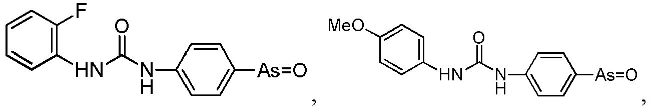

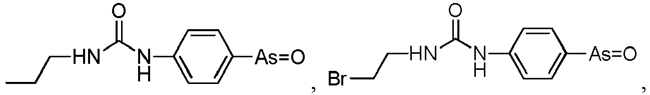

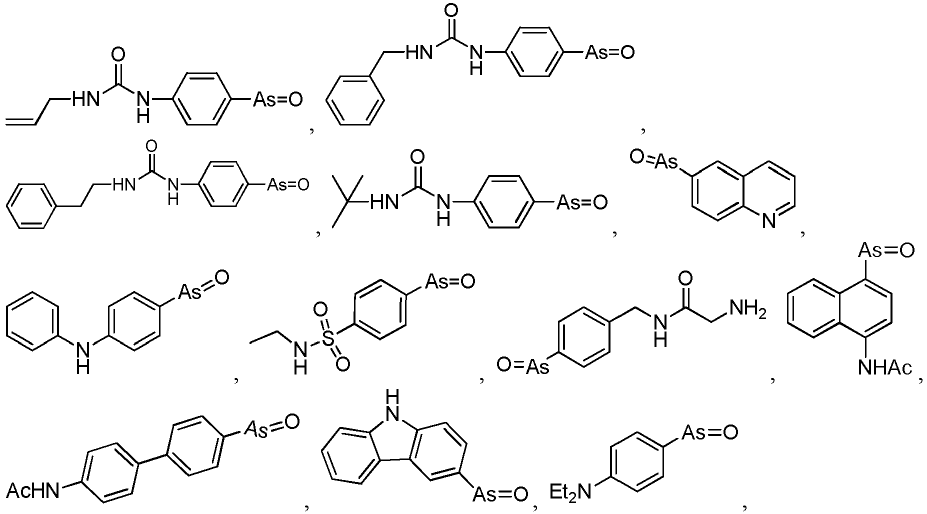

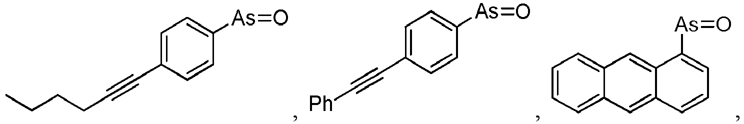

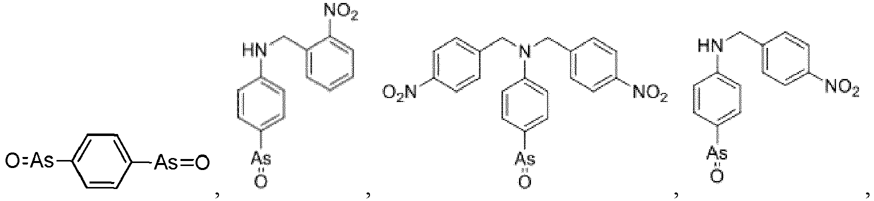

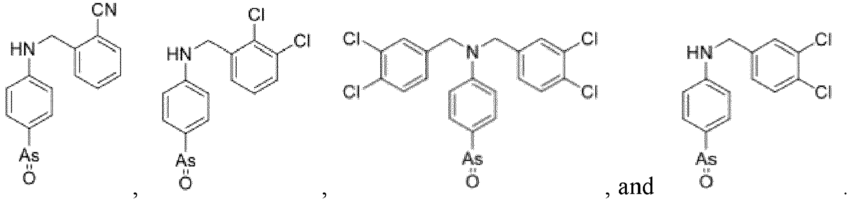

The term "phenylarsine oxide" (PAO) as used herein refers to a small molecule compound having a specific chemical structure shown as follows:

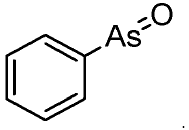

The terms "A1" and "G1" as used herein are both small molecule compound inhibitors of PI4KIIIα protein and have relatively similar structures. The chemical structural formula of A1 is: 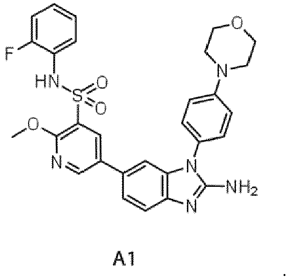 5-(2-amino-1-(4-(4-morpholinyl)phenyl)-1*H*-benzimidazol-6-yl)-*N*-(2-fluorophenyl)-2-methoxy-3-pyridinesulfonamide

The chemical structural formula of G1 is: 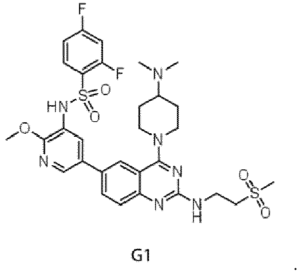 (a*S*)-5-(2-amino-4-oxo-3-(2-(trifluoromethyl)phenyl)-3,4-dihydroquinazolin-6-yl)-*N-*(2,4-difluorophenyl)-2-methoxypyridine-3-sulfonamide.

The present invention also relates to a derivative of phenylarsine oxide and an analog of A1 or G1, the derivative and the analog can also be used for treating intracellular protein misfolding-related diseases and lysosomal storage diseases, as long as the derivative and the analog has a function of inhibiting the phosphokinase activity of PI4KIIIα protein, and a method for preparing such structural analogs have also been disclosed. In some embodiments, the derivative of phenylarsine oxide, A1 or G1 is an analog with a structure similar to phenylarsine oxide, A1 or G1.

### Intracellular protein misfolding-related diseases

The term "intracellular protein misfolding-related disease" as used herein refers to a disease characterized by aggregation of abnormally folded proteins in the cytoplasm, and is also diagnosed as a protein aggregation/accumulation or protein misfolding disease. In addition, the term "intracellular protein misfolding-related diseases" also includes some intracellular inclusion body diseases such as protein inclusion body accumulation disease, in which inclusion body is mainly composed of a core protein aggregated due to folding error and various stress proteins which are involved in response to unfolded proteins and are externally attached.

Intracellular protein misfolding-related diseases include, but are not limited to, Parkinson's disease (PD), Lewy body dementia (LBD), multiple system atrophy (MSA), inclusion body myositis (IBM), frontotemporal dementia (FTD), Hungtington disease (HD), polyglutamine disease (PolyQ), amyotrophic lateral sclerosis (ALS), and Prion disease.

### Lysosomal storage diseases

The term "lysosomal storage disease" as used herein refers to a disease caused by accumulation of some endogenous or exogenous substances in lysosomes due to various causes, including but not limited to lysosome function defects caused by insufficient enzymatic activity in lysosomes, deficiency of activator protein, transporter protein or enzymes for lysosome protein processing and correction, which results in the failure of digestion of corresponding substrates in secondary lysosomes, accumulation of substrates and metabolic disorder, thus leading to storage diseases and the like.

Lysosomal storage diseases include, but are not limited to, sphingolipid metabolism disorder, mucopolysaccharidosis, glycogen storage disease, glycoprotein storage disease, lipid storage disease, post-translational modification deficiency, integral membrane protein deficiency disorder, neuronal ceroid lipofuscinosis, or lysosome-related organelle disorder. The sphingolipid metabolism disorder includes, but is not limited to, Fabry disease, metabolic disorder skin disease (Farbe disease), Gaucher disease types I, II, III and prenatal death, GM1 gangliosidosis types I, II and III, GM2 gangliosidosis (familial amaurotic idiocy), GM2 gangliosidosis, spheroid leukodystrophy (Krabbe disease), metachromatic leukodystrophy, and Niemann-Pick disease types A and B; mucopolysaccharidosis includes, but is not limited to, Hurler-Scheie syndrome and Scheie syndrome (ML I), Hunt syndrome (MPS II), Sanfilippo syndrome A (MPS IIIA), Sanfilippo syndrome B (MPS IIIB), Sanfilippo syndrome C (MPS IIIC), Sanfilippo syndrome D (MPS IIID), eccentro-osteochondrodysplasia syndrome (MPS IVA), eccentro-osteochondrodysplasia syndrome (MPS IVB), maroteaux-lamy syndrome (Lamy syndrome, MPS VI), Sly disease (MPS VII), and MPS IX; glycogen storage disease includes, but is not limited to, Pompe disease (GSD II); glycoprotein storage disease includes, but is not limited to, α-mannoside storage disease, β-mannoside storage disease, fucosidosis, aspartylglucosaminuria, Schindle disease type I (infantile onset neuroaxonal dystrophy), Schindle disease type II (Kanzaki disease), Schindle disease type III (moderate severity), Sialic acid storage disease type I (Cherry-red spot-myoclonus syndrome), Sialic acid storage disease type II (mucopolysaccharidosis I), and galactosialidosis; lipid storage disease includes, but is not limited to, acid lipase deficiency such as Wolfman disease and cholesteryl ester storage disease; post-translational modification deficiency includes, but is not limited to, multiple sulfatase deficiency, Mucolipidosis II α/β (I-cell disease), Mucolipidosis II α/β (Pseudo-Hurler syndrome), and Mucolipidosis III γ (Pseudo-Hurler syndrome variant); integral membrane protein deficiency disorder includes, but is not limited to, Cystinosis, Danon's disease, myoclonus renal failure syndrome, Sialic acid storage disorders such as ISSD, Salla syndrome and moderately severe Salla syndrome, Niemann-Pick disease types C1 and C2, and Mucolipidosis type IV; neuronal ceroid lipofuscinosis include, but is not limited to, ceroid lipofuscinosis type 1 (Haltia-Santavuori syndrome and INCL), neuronal ceroid lipofuscinosis type 2 (Jansky-Bielschowsky syndrome), ceroid lipofuscinosis type 3 (Batten-Spielmeyer-Sjogren syndrome), ceroid lipofuscinosis type 4 (Parry's disease and Kufs types A and B), ceroid lipofuscinosis type 5 (late infant Finnish variant), ceroid lipofuscinosis type 6 (Lake-Cavanagh or Indiana variant), ceroid lipofuscinosis type 7 (Turkish variant), ceroid lipofuscinosis type 8 (northern epilepsy, epileptic intellectual deterioration), ceroid lipofuscinosis type 9, ceroid lipofuscinosis type 10, ceroid lipofuscinosis type 11, ceroid lipofuscinosis type 12, ceroid lipofuscinosis type 13, ceroid lipofuscinosis type 14; and lysosome-related organelle disorder includes, but is not limited to, Hermansky-Pudlak disease type 1, Hermansky-Pudlak disease type 2, Hermansky-Pudlak disease type 3, Hermansky-Pudlak disease type 4, Hermansky-Pudlak disease type 5, Hermansky-Pudlak disease type 6, Hermansky-Pudlak disease type 7, Hermansky-Pudlak disease type 8, Hermansky-Pudlak disease type 9, Griscelli syndrome 1 (Elejalde syndrome), Griscelli syndrome 2, and Chédiak-Higashi disease.

### Drug administration and medical use

The term "pharmaceutically acceptable" as used herein refers to those compounds, materials, compositions, and/or dosage forms that are suitable for use in contact with human and animal tissues within the scope of reasonable medical judgment without excessive toxicity, irritation, allergic response, or other problems or complications, and are commensurate with a reasonable benefit/risk ratio. In certain embodiments, pharmaceutically acceptable compounds, materials, compositions, and/or dosage forms refer to those approved by a regulatory agency (e.g., the United States Food and Drug Administration, the National Medical Products Administration of China, or the European Medicines Agency) or listed in a generally recognized pharmacopeia (e.g., *U.S. Pharmacopeia, Chinese Pharmacopoeia,* or *European Pharmacopoeia)* for use in animals (more particularly in humans).

The term "subject" as used herein may include both humans and non-human animals. Non-human animals include all vertebrates, for example, mammals and non-mammals. The "subject" can also be livestock animals (e.g., cows, pigs, sheep, chickens, rabbits or horses), or rodents (e.g., rats or mice), or primates (e.g., gorillas or monkeys), or domestic animals (e.g., dogs or cats). The "subject" may be male or female, or may be of different age. The human "subject" may be Caucasian, African, Asian, Semitic, or other races, or a hybrid of different races. The human "subject" may be the elderly, adults, teenagers, children or infants.

In some embodiments, the subjects described herein are humans or non-human primates.

The PI4KIIIα specific inhibitors disclosed herein can be administered by an administration route well known in the art, such as by injection (e.g., subcutaneous injection, intraperitoneal injection, intravenous injection (including intravenous drip or intravenous infusion), intramuscular injection or intradermal injection) or non-injection (e.g., oral, nasal, sublingual, rectal, or topical administration). In some embodiments, the PI4KIIIα specific inhibitor described herein is administered orally, subcutaneously, intramuscularly or intravenously. In some embodiments, the PI4KIIIα specific inhibitor described herein is administered orally.

The term "therapeutically effective amount" as used herein refers to an amount of a drug that alleviates or eliminates a disease or a symptom of the subject, or that prophylactically inhibits or prevents the onset of a disease or a symptom. A therapeutically effective amount may be an amount of a drug that will alleviate to some extent one or more diseases or symptoms of the subject; an amount of a drug that can partially or completely restore to normal one or more physiological or biochemical parameters associated with the cause of a disease or a symptom; and/or an amount of a drug that may reduce the likelihood of the development of a disease or a symptom. In some embodiments, the term "therapeutically effective amount" as used herein refers to an amount of a drug that can alleviate or eliminate intracellular protein misfolding-related diseases or lysosomal storage diseases of a subject.

The therapeutically effective dose of the PI4KIIIα specific inhibitor provided herein depends on various factors well known in the art, such as body weight, age, past medical history, current treatment underway, the health status of the object and the strength of drug interactions, allergies, hypersensitivity and side effects, as well as administration routes and disease progression degree. One skilled in the art (e.g., a physician or veterinarian) can lower or raise the dose accordingly to these or other conditions or requirements.

In some embodiments, the treatment further comprises administering a second agent to a subject in need thereof.

In some embodiments, the second agent is an agent for treating intracellular protein misfolding-related diseases, including but not limited to Levodopa and Riluzole.

In some embodiments, the PI4KIIIα specific inhibitor is administered prior to, subsequent to or concurrently with the second agent.

The present application also relates to a method for preventing or treating intracellular protein misfolding-related diseases comprising administering an effective amount of a PI4KIIIα specific inhibitor to a subject in need thereof.

The present application also relates to a method for preventing or treating lysosomal storage diseases comprising administering an effective amount of a PI4KIIIα specific inhibitor to a subject in need thereof.

### Drug screening

The present invention provides a method for screening a drug for preventing or treating intracellular protein misfolding-related diseases, comprising contacting a drug candidate with a PI4KIIIα protein or nucleic acid or PI4KIIIα, and detecting whether the drug candidate is capable of inhibiting the formation or activity of PI4KIIIα.

The present invention further provides a method for screening a drug for preventing or treating lysosomal storage diseases, comprising contacting a drug candidate with a PI4KIIIα protein or nucleic acid or PI4KIIIα, and detecting whether the drug candidate is capable of inhibiting the formation or activity of PI4KIIIα.

### Example 1. Promotion of Exocytosis of Microglia Lysosomes by PI4KIIIα Inhibitors

### 1.1 Experimental animals

The mice used were wild type (WT) C57BL/6J mice and C57BL/6J mice with heterozygous knockout (KO) of PI4KA. Male PI4KA^{-/+} mutant mice [Pi4kaGt (RRO073) Byg/+, MMRRC, Cat. #016351, UC Davis Mouse Resource Center (USA)] and wild type C57BL/6 mice backcrossed for more than 5 generations. The resulting Pi4kaGt (RRO073) Byg/+ mice were then used in this study. WT mice were purchased from GemPharmatech Co., Ltd., and all mice were bred at the animal research center of the Institute of Pharmacy, Fudan University (Shanghai, China). All mice were kept in standard cages of no more than 5 mice/cage, with no restriction of food and water intake during the 12 hour of light/dark cycle. For mice requiring breeding, no more than one male and two female per cage were kept for breeding.

### 1.2 Isolation and culture of primary microglia (complete medium: MEM + 10% FBS)

### a. Isolation and culture of mixed microglia

Pregnant mice for 18-20 days were anesthetized with anesthetic (1.2% Abamectin injected into abdominal cavity at 0.3 mL/10 g), embryos were collected and placed into a sterilized beaker, the mouth of the beaker was sealed with a thin film glove and transferred to a cell room. Heads were cut, brains were taken off, and the previous steps were consistent with the that of taking primary neuronal cells. The taken brain tissue was placed in a cell culture dish containing Hank's balanced salt solution (HBSS), and then cerebral cortex was separated under an anatomic microscope and placed in HBSS, and meninges and blood vessels attached to the cerebral cortex were peeled off, torn up with forceps, and sucked into a 15 mL EP tube. After the HBSS washing, HBSS was discarded, trypsin was added (the trypsin volume depended on the cell amount, generally 10× trypsin ratio was 1 : 10), and the mixture was digested at 37°C for 10 minutes in a CO₂ incubator, then the trypsin was discarded, and FBS (about 20% of the total volume) was added to terminate the digestion reaction for 1.5 to 2 minutes. FBS was carefully pipetted by using a disposable pipette, MEM culture solution was added, the mixture was repeatedly pipetted until the culture solution was suspension containing cells, at this time, the cells were uniformly dispersed in the MEM culture solution without obvious lumps and tissue fragments and sieved by using a 70-micron cell sieve; complete culture medium was added, the cells were uniformly pipetted followed by counting; MEM complete culture medium containing 10% of FBS was added into T25 cell culture flasks, and microglial cells separated from the brain of one fetal mouse were cultured in each one culture flask. The culture flasks were incubated in an incubator with CO₂, and the old culture medium was discarded after overnight and replaced with fresh culture medium; after that, the medium was refreshed every 48 hours, the layered growth of cells was observed after about 10 days, wherein the upper layer with better refractivity was microglia, and the lower layer supporting growth was astrocytes.

### b. Separating and purifying microglia by mild trypsin digestion method

The old culture medium was discarded, PBS was used for washing three times and 5 mL of low-concentration 0.125% trypsin was added for digestion, and the culture flask was observed under an inverted microscope while shaking; the suspension containing the microglia was transferred into a 15 mL centrifuge tube after most of the upper layer cells were detached and centrifuged at the low temperature of 4°C for 5 minutes at the rotating speed of 1000 g, the supernatant was removed by pipetting, then 2 mL of complete culture medium was added to resuspend the cell precipitate, the cell suspension was mixed well with 0.4% trypan blue solution in a ratio of 9 : 1, and then the mixture was added dropwise to a blood counting chamber for counting. After counting, the cells were inoculated in a polylysine-coated 35 mm glass-bottom culture dish at a cell count of 1 × 10⁶, and after 30 minutes, the old medium was replaced with a fresh complete medium, the non-completely attached promiscuous cells were removed and the remaining cells were cultured in an incubator with CO₂. The medium was replaced with a fresh complete culture solution 24 hours after purification, and then all of or half of the culture solution was replaced every 2 days. After the primary microglia was cultured for at least 4 hours, the medium was replaced with the serum-free culture medium, and then subsequent experiments were performed.

### 1.3 Immunol staining of microglia Iba1 antibody

The Iba1 (Ionized calcium-binding adapter molecule 1) antibody is a specific antibody of microglia and macrophages, can specifically bind to calcium binding protein specifically expressed by the microglia, and is a marker of the microglia in the central nervous system.

The formulation of 4% (w/v) of PFA: Firstly, 700 mL of water was stirred under magnetic force to accelerate temperature rise, a few drops of 1M sodium hydroxide solution was added to assist dissolution after temperature rise, then 40 g of paraformaldehyde (PFA) was added and stirred to be completely dissolved, 100 mL of heated 10× PBS was added to the solution, the volume thereof was made up to 1 L with water, the pH value was adjusted to 8.0, the mixed solution was filtered and aliquoted into 50 mL centrifuge tubes, and the centrifuge tubes were placed in a refrigerator at 4°C to be stored after being cooled.

The separated and purified microglia were seeded into a 12-well plate paved with a cover glass coated by PDL, when the cell confluence reached about 70%, the culture medium was discarded and PBS was used for washing three times (5 minutes for each time), the PBS was discarded, 4% (w/v) of PFA was used for fixation at 4°C for 20 minutes and PBS was used for washing three times (5 minutes for each time), and then 0.2% (w/v) of Triton X-100-PBS was used for permeabilization at room temperature for 5 minutes and PBS was used for washing three times (5 minutes for each time). The fixed cells were blocked by incubation with 10% BSA (PBS) for 1 hour at room temperature and washed twice with PBS. The cell slide was placed in a dark box and incubated overnight at 4°C with anti-Iba1 antibody, washed three times with PBS (5 minutes for each time). Fluorescence-labeled secondary antibody was added and incubated in the dark for 1 hour at room temperature, and PBS was used for washing four times (5 minutes for each time). Anti-fluorescence quenching mounting solution containing DAPI dye was added, the cover glass was reversely buckled on the glass slide, and the cell slide was sealed with transparent nail polish and placed in a dark box for assay. The primary antibody was diluted at 1 : 500, and the secondary antibody was diluted at 1 : 1000.

3 fluorescence pictures were randomly selected and the entire picture was analyzed using Image-pro-plus software. The red spot was the number of positive cells, the blue spot was the total number of nuclei, and the positive cell rate was calculated. Calculation formula: Positive cell rate (%) = the number of positive stained cells/total number of nuclei × 100%. The purity of the purified microglia in the experiment was > 95% after the positive cell rate was calculated, and the purified microglia can be used for subsequent experiments.

### 1.4 Fluorescent staining and living-cell imaging

Primary microglia requiring live-cell imaging were inoculated in a glass-bottom culture dish, and the old culture medium was replaced with a serum-free culture medium for overnight culture before the experiment. 1 mM LysoTracker Red DND-99 was diluted to 50 nM in MEM complete medium containing 10% FBS; after the old culture medium was replaced with the loading solution containing the fluorescent probe, the culture dish was placed in a CO₂ incubator for incubation for 30 minutes to stain lysosomes of the living cells, and intracellular ATP was stained with Quinacrine diluted to 100 nM in complete medium. Fluorescence pictures were taken with a Nikon inverted microscope (Nikon Eclipse Ti-E, Japan) using a Plan Apo 60 × 1.40-fold oil lens as objective lens, real-time live cell imaging was performed by continuous laser scanning, wherein the emission of LysoTracker was 561 nm, and the emission of Quinacrine was 488 nm. High-concentration PAO mother liquor was added into a cell culture medium in a spotting manner to enable the final concentration of the PAO mother liquor in the culture medium to be 100 nM, timing was performed from the 0^{th} second of PAO addition in spotting, a fluorescence image was collected every 10 seconds by time lapse shooting, wherein the total shooting time was 500 seconds, and fluorescence image analysis was performed by using Volocity software (PerkinElmer). The laser power used in the time lapse shooting was reduced as much as possible (< 1%) to avoid possible fluorescent bleaching.

### 1.5 Treatment of cells by PI4KIIIα inhibitor

Primary microglia were treated with phenylarsine oxide (PAO) which was a specific inhibitor of PI4KIIIα at a low concentration. Primary microglia cultures were incubated with PAO at concentrations of 0, 25 or 100 nM diluted with complete medium for 1 hour, respectively, and then cell samples were collected for lysosomal exocytosis imaging or culture solution was collected for determining ATP concentration in extracellular fluid. Microglia were treated with MEM culture solution containing 10% FBS without PAO as a control experiment.

### 1.6 Determination of ATP concentration in cell culture solution

The content of ATP in extracellular fluid was determined by bioluminescence method using a luciferase reporter experiment. Microglia were starved in serum-free MEM medium overnight. In order to prevent ATP hydrolysis, the ATP hydrolase inhibitor Dipyridamole (10 µM) was added to the medium throughout the experiment. 0, 25, 100 nM of PI4KIIIα inhibitor PAO was added to the medium, respectively, and the medium was collected after incubation for 0, 4, 6, 8, 15, 30, and 60 minutes, respectively. 50 µL of the ATP assay mixture containing luciferase-luciferin buffer was added to an opaque white 96-well plate, the plate was allowed to stand for two minutes to consume ATP in the wells, and then 50 µL of the sample was added to the wells. The luminescence value of the solution was measured using a Varioskan Flash (Thermo Fisher Scientific) multifunctional microplate reader. A standard curve was obtained from the luminescence value of the standard ATP sample, and the content of ATP in the sample was obtained by reference to a standard equation. Data were presented as mean ± SEM of at least 3 experiments per group. Statistical data analysis was performed using one-way ANOVA analysis.

### 1.7 Determination of total protein content in cells

Old cell culture medium was aspirated and PBS buffer was added for washing 3 times, PBS was discarded, and RIPA lysate containing 1% protease inhibitor (100 µL/well in 12-well plate) was added, the plate was placed on ice for lysis for 30 minutes, and then the cells at the bottom of the wells were scraped with a cell scraper and placed in a 1.5 mL centrifuge tube, followed by centrifugation at 15000 rpm for 30 minutes at 4°C to collect supernatant and determine the total protein content therein. A working solution and a standard solution were formulated according to the requirements of the specification, the sample and the working solution were added into a 96-well plate in a ratio of 1 : 8, that is, 25 µL of the sample, BSA standard solution and 200 µL of the working solution were added into each well, the mixture was uniformly mixed on a flat plate oscillator for 30 seconds, then the well plate was covered with an aluminum foil and incubated for 30 minutes at 37°C, and the absorbance at 562 nm of the well plate was measured after the well plate was cooled to room temperature. The total intracellular protein concentration can be calculated from a standard curve.

### 1.8 Statistical analysis

Data processing was analyzed using GraphPad Prism 5 software. One-way ANOVA analysis was used for treatment, mean ± SEM, p < 0.03 indicates that there was a statistical difference.

### 1.9 Promotion of decrease of microglial lysosomal fluorescence intensity by inhibiting PI4KIIIα

The purity of the purified microglia in the experiment was > 95% after the positive cell rate was calculated, and the purified microglia can be used for subsequent experiments.

For cultured microglia (from C57B6 or PI4KA^{-/+} mutant mice, respectively), 1 mM LysoTracker Red DND-99 was diluted to 50 nM, and after the old culture medium was replaced with the loading solution containing the fluorescent probe, the culture dish was incubated for 30 minutes to stain lysosomes of the living cells. After the primary microglia cell cultures were cultured with PAO at 100 nM concentration or culture medium for 1 hour, fluorescence imaging was performed (FIG. 1).

FIG. 1 shows that the fluorescence intensity decreases with time (from 0 to 500 seconds), indicating that the fusion of lysosomal membrane with cytoplasmic membrane causes dissociation of the fluorescent dye within the lysosomes. In the control group (wild type WT mouse cells without PAO) of FIG. 1(A), the fluorescence intensity of each fluorescent spot did not change much within 500 seconds, and the fluorescence intensity decreased by 13.5% (p < 0.001) at 250 seconds and by only 15.1% (p < 0.001) after the conventional 500-second lysosomal exocytosis, as compared to the fluorescence intensity at 0 seconds. In the PAO (100 nM) group of FIG. 1 (B), 5.2% discoloration (p < 0.001) was shown at 100 seconds, 47.4% discoloration (p < 0.001) was achieved at 200 seconds, 68.5% (p < 0.001) at 400 seconds, and 70.1% (p < 0.001) at 500 seconds. In the PI4K+/- group of FIG. 1(C), the decrease in fluorescence intensity was insignificant for some spots, the decrease in mean fluorescence intensity was 45.1% (p < 0.001), and the magnitude of the change in fluorescence intensity decreased after 200 seconds.

### 1.10 Decrease of lysosomal fluorescence in microglia by BPB

In order to further validate this "kiss-and-run" fusion format, a membrane-impermeable fluorescence quencher, bromophenol blue (BPB), was introduced at a working concentration of 1 mM. BPB can diffuse rapidly through the fusion pore formed during the "kiss-and-run" process to quench residual fluorescence on the leaflets inside the lysosome membrane. Primary microglia without any treatment could undergo regular exocytosis, allowing a small amount of BPB to enter the cell, while when PI4KIIIα enzyme activity was inhibited, a large amount of BPB entered inner lobe of lysosome through the fusion pore produced in the "kiss-and-run" process, causing a brief quenching of intracellular lysosomal fluorescence (FIG. 2).

FIG. 2(A) shows a discoloration of 17.7% (p < 0.001) at 250 seconds and 20.2% (p < 0.001) at 500 seconds, as compared to the fluorescence intensity at 0 seconds, in the control group. FIG. 2(B) shows a discoloration of 22.3% (p < 0.001) at 10 seconds, 55.1% (p < 0.001) at 50 seconds, 62.8% (p < 0.001) at 100 seconds, 69.1% (p < 0.001) at 200 seconds, 75.5% (p < 0.001) at 400 seconds, and 78% (p < 0.001) at 500 seconds, as compared to the fluorescence intensity at 0 seconds after PAO treatment. In FIG. 2(C), the mean fluorescence intensity of 22 fluorescent spots was reduced by 62.5% at 500 seconds as compared to mean fluorescence intensity at 0 seconds.

### 1.11 Recovery/increase of microglial lysosomal fluorescence caused by dispersion of BPB out from lysosomes

In addition, when incubation was performed in BPB solution for 1 hour, BPB entered cells through conventional exocytosis to reach a limited concentration, so that lysosomal fluorescence was quenched; at this time, down-regulation of PI4KIIIα enabled "kiss-and-run" fusion of the lysosomes with the cytoplasmic membrane, and BPB diffused out of the lysosomes before the lysosomal dye dissociated; at this time, the lysosomal fluorescence that was temporarily quenched re-emitted (FIG. 3).

FIG. 3(A) is a control experiment where the lysosomal fluorescence intensity was almost unchanged at 500 seconds, but slightly increased by 1.4% as compared to the fluorescence intensity at 0 seconds. In FIG. 3(B), in the first 200 seconds, due to the presence of fusion pore, the extracellular BPB entered the WT cells to temporarily quench the lysosomal fluorescence, with an average decrease in fluorescence intensity of 41.8% compared to 0 seconds. In the next 300 seconds, due to the presence of the fusion pore, the intracellular BPB was again discharged from the cell through the fusion pore before the dye dissociated from the lysosome, allowing the recovery of the briefly quenched fluorescence, with an increase of 26.4% compared to 0 seconds. In FIG. 3(C), intracellular and extracellular BPB concentrations of PI4K^{-/+} heterozygous knockout cells after BPB incubation for 1 hour reached equilibrium because lysosomes were always in the "kiss-and-run" state during this process, eventually with an average increase in fluorescence intensity of 11.7% at 500 seconds as compared to 0 seconds.

### 1.12 Promotion of extracellular ATP secretion of microglial lysosomes by PI4KIIIα inhibitor

It has been reported in literature that extracellular ATP played an important role in promoting the fusion of lysosomes with cytoplasmic membranes, and it had been verified that a large amount of ATP accumulated in microglia (Dou Y, Wu H J, Li H Q, et al. Microglial migration mediated by ATP-induced ATP release from lysosomes [J]. Cell Research, 2012, 22(6): 1022-1033; Imura Y, Moriza WA Y, Komatus R, et al. Microglia release ATP by exocytosis [J]. Glia, 2013, 61(8): 1320-1330; Zhang Z, Chen G, Zhou W, et al. Regulated ATP release from astrocytes through lysosome exocytosis [J]. Nature Cell Biology, 2007, 9(8):945-953). Inhibition of PI4KIIIα promoted "kiss-and-run" fusion of microglial lysosomes with the cell membrane, so as to release ATP from the lysosomes. The ATP was released extracellularly to further promote the fusion of lysosomes with cell membrane, so as to release the cell degradation products and cytotoxic substances in lysosomes, such as β amyloid. In order to verify accumulation and release of ATP in lysosomes, the present invention used 100 nM Quinacrine to label intracellular ATP (FIG. 4).

In FIG. 4, it could be seen from the merged graphs that intracellular ATP was co-localized with lysosomes, which verified that a large amount of ATP was accumulated in the microglial lysosomes. At this time, the PAO was incubated, ATP was released extracellularly and intracellular ATP was reduced. As can be seen from the data analysis in FIG. 4(B), the extracellular ATP concentrations measured at 8, 15, 30, and 60 minutes in PAO-treated WT cells and PI4K^{+/-} cells were significantly increased compared with the control group (p < 0.05).

### Example 2. Promotion of Exocytosis of Neuronal Lysosomes by PI4KIIIα Inhibitors

### 2.1 Isolation and culture of primary hippocampus neuronal cells

Pregnant mice for 18-20 days were anesthetized with anesthetic (1.2% Abamectin injected into abdominal cavity at 0.3 mL/10 g), embryos were collected and placed into a sterilized beaker, the mouth of the beaker was sealed with a thin film glove and transferred to a cell room. The head was cut, the brain was taken, the taken brain tissue was placed in a cell culture dish containing DMEM high-glucose culture medium, and then hippocampus was separated under an anatomic microscope and placed in DMEM high-glucose culture medium, and meninges and blood vessel attached to hippocampus were peeled off, torn up with forceps, and sucked into a 12-well plate. After DMEM high-glucose medium was added for rinse thoroughly, the DMEM high-glucose medium was carefully aspirated by a micropipette, and then the plate was added with 1 mL of 0.125% trypsin, and placed in an incubator with carbon dioxide at 37°C for gentle digestion for 8 minutes; the plate was taken out every 4 minutes and shaken to ensure uniform digestion, the trypsin was carefully aspirated and discarded, and fetal bovine serum (FBS) (about 20% of the total volume) was added to stop the digestion reaction for 1.5-2 minutes. The FBS was carefully aspirated, and inoculum (DMEM-HG), was added, pipetted 10 times and then allowed to stand for 2 minutes; free single cells were in the supernatant, lumps were deposited on the bottom, the supernatant was transferred to an ice-bath culture dish, and the deposited lumps were discarded after the above operations were repeated three times. The cells were counted after the cells were pipetted uniformly, each well of the blood counting plate needed to be counted, and if the cells were not uniformly distributed, the cells needed to be counted again. After counting was completed, inoculum was added to uniformly pipette cells, and the cells were seeded in a cell culture plate (typically 6-well plate at 1^{∗}10⁵ cells/well, and so on, the number of cells were increased as appropriate if transfection was required). The plate was placed in an incubator with CO₂ for incubation for 4 hours, then the old medium was replaced with the neurobasal culture medium, the liquid in the plate was slightly shaken during medium replacement, and the plate was washed with inoculum at 37°C to detach loosely attached vascular cells. Thereafter, half of the medium was changed every two days, and culture was performed *in vitro* for 10 days (DIV10) for subsequent experiments.

### 2.2 Promotion of decrease of lysosomal fluorescence intensity of neuronal cells by inhibiting PI4KIIIα

Primary hippocampal neurons were isolated from the hippocampus of WT embryonic mice, and PI4KIIIα drug inhibition was performed on the primary neuronal cells, and lysosomal changes in the drug treated wild-type neuronal cells and non-drug treated wild-type neuronal cells were compared, respectively (FIG. 5).

FIG. 5(A) shows that the number of lysosome fluorescent spots hardly changes in WT primary neuronal cells without inhibiting PI4KIIIα activity by PAO. FIG. 5(B) shows that lysosomal fluorescence intensity significantly decreased compared to control group after incubation with PAO solution in wild type primary neuronal cells.

### Example 3. Inhibition of Decrease in Intracellular α-Synuclein Protein Levels by PI4KIIIα Inhibitors

### 3.1 Culture of PC12, SH-SY5Y cell lines

The PC12 complete culture system was prepared by adding 10% HS and 5% FBS into high-glucose DMEM; and SH-SY5Y complete culture system was prepared by adding 15% FBS in high-glucose DMEM.

### 3.2 Immunofluorescent staining of PC12 and SH-SY5Y cells

After the cell culture systems were treated by the drug, the following operations was performed, fixation: treating with 4% PFA for 30 minutes; permeabilization: treating with 0.1% Triton-X for 15 minutes; and blocking: blocking with 10% donkey serum for 1 hour; primary antibody: mouse anti-α-synuclein; secondary antibody: anti-Mouse Alex 555.

### 3.3 α-synuclein ELISA assay

50 ul Hu α-synuclein Detection Antibody solution was added to each well (except for chromogen blanks empty, that is, blank colorimetric well), 50 ul sample and standard curve regent (prepared in the following figure) were added to each well (except for chromogen blanks empty), and gentle shaking was performed for homogeneous mixing, and a film was used for covering wells and incubation was performed overnight at 4°C; after the wells were fully washed four times with 100 ul of 1X Wash buffer, each well was added with 100 ul of Anti-Rabbit IgG HRP except the chromogen blanks empty, a film was used for covering wells and incubation was performed for 30 minutes at room temperature, and the wells were fully washed four times with 1× Wash buffer again; each well was added with 100 µL of Stabilized Chromogen, the solution turned blue, and the wells were incubated for 30 minutes at room temperature in a dark place; and each well was added with 100 µL of Stop Solution. The mixture was mixed uniformly by gentle shaking, the solution turned yellow, and read was performed with a microplate reader (wavelength: 450 nm).

### 3.4 Treatment of cells by PI4KIIIα inhibitor

PC12 or SY cell culture systems: 10% HS + 5% FBS + high-glucose DMEM, culturing to 40-60% culture dishes, and Fugene HD transfection reagent was used to transfect α-synuclein over-expression plasmid. After 24 hours of culture, starvation culture was performed for 24 hours (serum was removed), and the culture system was changed to the normal culture system, and Lyso-tracker was added for treatment for 30 minutes, followed by fresh culture medium replacement and PAO treatment. PAO treated group: Immunocytochemical staining was performed after treatment for 10 minutes at 25 nM, 50 nM, 100 nM.

### 3.5 Determination of cell viability by Thiazolyl blue (MTT) assay

The drug was administrated for treatment for 12 hours, MTT at a final concentration of 05. mg/mL was added, the culture solution was aspirated after 4 hours of incubation, 100 µL of DMSO was added to dissolve the adsorbed MTT, and the absorbance value was read after 15 minutes of shaking.

### 3.6 Plasmid transfection

The information of the α-synuclein over-expression plasmid was as follows:

ATGGATGTATTCATGAAAGGACTTTCAAAGGCCAAGGAGGGAGTTGTGGCTGCTG
CTGAGAAAACCAAACAGGGTGTGGCAGAAGCAGCAGGAAAGACAAAAGAGGGT
GTTCTCTATGTAGGCTCCAAAACCAAGGAGGGAGTGGTGCATGGTGTGGCAACA
GTGGCTGAGAAGACCAAAGAGCAAGTGACAAATGTTGGAGGAGCAGTGGTGACG
GGTGTGACAGCAGTAGCCCAGAAGACAGTGGAGGGAGCAGGGAGCATTGCAGCA

GCCACTGGCTTTGTCAAAAAGGACCAGTTGGGCAAGAATGAAGAAGGAGCCCCA
CAGGAAGGAATTCTGGAAGATATGCCTGTGGATCCTGACAATGAGGCTTATGAA
ATGCCTTCTGAGGAAGGGTATCAAGACTACGAACCTGAAGCCTAA (SEQ No. 4)

The α-synuclein ELISA Kit was purchased from Thermo Fisher Scientific (Catalog No. KHB0061). Fugene HD transfection reagent was purchased from Promega (Beijing) Biotech Co., Ltd. (Catalog No. E2311); and α-synuclein monoclonal antibody (Mouse monoclone) was purchased from Sigma-Aldrich (Shanghai, Cataltlog No. S5566).

### 3.7 PAO inhibition of cell death or apoptosis caused by over-expression of α-synuclein in SH-SY5Y cells

The hallmark cytopathological feature of Parkinson's disease patients is the occurrence of protein aggregation dominated by misfolded α-synuclein proteins. These aggregated proteins are thought to cause cytotoxicity because they cannot be cleared in time. The present invention transfected SH-SY5Y cell line with α-synuclein over-expression (α-syn-OE) plasmid, and after 24 hours, PAO treatment was given for 12 hours. Cell viability rate was then determined for each group by MTT assay and compared to the α-syn-OE group (FIG. 6).

After One-way ANOVA analysis, the results show as follows: FIG. 6(A) shows that cell viability rate of α-syn-OE group was significantly lower than that of the control (Vehicle) group; and FIG. 6(B) shows that cell viability rate of the α-syn-OE + PAO treated group (PAO at concentrations of 6.25, 12.5, 25, 50, 75, 100 nM, respectively) was significantly higher than that of the α-syn-OE group (n = 5, mean ± SEM, One-way ANOVA, **p < 0.01, ^{∗∗∗}p < 0.0001 vs. α-syn-OE). This experiment demonstrates that PAO can act to alleviate cytotoxicity caused by over-expression of α-synuclein.

### 3.8 Promotion of α-synuclein secretion by PAO series compounds

The present invention further assayed the characteristic of PAO for promoting the secretion or excretion of α-synuclein. The cultured PC12 and SH-SY5Y cell lines were transfected with α-synuclein over-expression plasmids by using Fugene HD transfection reagents, respectively, the old culture system was replaced with a complete culture system after starvation treatment was performed for 24 hours, meanwhile, the compound PAO or PAO derivative *p*-methyl phenyl arsine oxide was used for treatment for 4 hours, and the α-synuclein level in the supernatant of the cell culture solution was determined by ELISA.

The PAO derivative p-methyl phenyl arsine oxide has the following chemical formula:

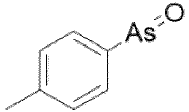

FIG. 7(A) shows that extracellular α-synuclein protein levels in the 50 nM, 75 nM and 100 nM PAO-treated groups were significantly increased compared to the control α-synOE group without PAO in the P12 cell line, suggesting that PAO was capable of promoting the efflux of intracellular α-synuclein protein. FIG. 7(B) shows that PAO had the same capability of promoting extracellular α-synuclein protein levels in SH-SY5Y cell line. FIG. 7(C) shows that 50 nM, 75 nM PI-70 treatment also promoted the efflux of intracellular α-synuclein protein in the P12 cell line.

### 3.9 Promotion of decrease in intracellular α-synuclein protein levels by PAO

The present invention firstly performed PAO treatment on cells, and then determined the intracellular α-synuclein protein level by an ELISA method and Western blot. The cell culture and drug treatment manner were the same as above. In FIG. 8, the results for ELISA assay showed that the α-synuclein protein levels were significantly reduced in the 75 nM and 100 nM PAO-treated groups compared to the α-syn OE group, suggesting that PAO was capable of reducing intracellular α-synuclein protein levels. In FIG. 8(B), the results for Western blot assay showed that α-synuclein in the 75 nM and 100 nM PAO-treated groups was lower than that in the control group.

### Example 4. Promotion of Secretion of HTT and SOD1 Proteins by PI4KIIIα Inhibitors

### 4.1 SOD1 ELISA assay

100 ul of the diluted standard curve regent, blank control and sample were added to the corresponding well of the assay well plate. The plate was covered with a film and incubated at 37°C for 2 hours; the liquid in each well was discarded, and each well was added with 100 ul Detection Reagent A Working Solution and the plate was covered with a film and incubated at 37°C for 1 hour; the supernatant was discarded, and each well was washed 3 times with 1X Wash buffer for 2 minutes each time with as no liquid residue as possible; each well was added with 100 ul Detection Reagent B Working Solution and the plate was covered with a film and incubated at 37°C for 1 hour; the washing operation was repeated 5 times; and each well was added with 90 µL of Substrate Solution and the plate was covered with a film and incubated for 20 minutes at 37°C in the dark. The solution turned blue; 50 µL of Stop Solution was added to each well, the mixture was mixed uniformly by gently shaking, the solution turned yellow, and read was performed with a microplate reader as soon as possible (absorption wavelength: 450 nm).

### 4.2 Human Huntingtin ELISA assay

50 ul of the diluted standard curve regent, blank control and sample were added to the corresponding well of the assay well plate; and 50 ul of Detection A Working Solution was immediately added to each well. After gently shaking for mixing uniformly, the plate was covered with a film and incubated at 37°C for 1 hour; the liquid in each well was removed, the supernatant was discarded, and each well was washed 3 times with 1X Wash buffer for 2 minutes each time with as no liquid residue as possible; 100 ul of Detection Reagent B Working Solution was added to each well. The plate was covered with a film and incubated for 45 minutes at 37°C, and the washing operation was repeated 5 times; and each well was added with 90 µl of Substrate Solution. the plate was covered with a new film and incubated for 10-20 minutes at 37°C in the dark; and 50 µl of Stop Solution was added to each well, the mixture was mixed uniformly by gently shaking, the solution turned yellow, and read was performed with a microplate reader immediately (absorption wavelength: 450 nm).

### 4.3 Transfection reagents

The transfection PC12 cell line was transfected with either HTT (1-586aa) (HTT (1-586aa) over-expression plasmid was capable of expressing the N-end of HTT protein which was an important part for HTT protein shuttling) or SOD1 over-expression plasmid using Fugene HD transfection reagent.

### 4.4 Plasmid transfection

The following plasmids were purchased from Obio Technology (Shanghai) Corp., Ltd:
1) Gene name: HTT (1-586aa); GenBank ID:NM_002111;
2) Gene name: SOD1, gen ID: 6647.

Human Superoxide Dismutase 1, Soluble (SOD1) ELISA Kit was purchased from Signalway Antibody LLC. (SAB, Catalog No. EK16688); Human Huntingtin (HTT) ELISA Kit was purchased from Signalway Antibody LLC. (SAB, Catalog No. EK2869).

### 4.5 Promotion of secretion of HTT and SOD1 proteins by PI4KIIIα Inhibitors

In neurological diseases, the HTT and SOD1 proteins are also closely related to the development of various neurological diseases, with Hungtington disease is related to HTT proteins and amyotrophic lateral sclerosis is related to SOD1 proteins. The PC12 cell line was transfected with either HTT (1-586aa) (HTT (1-586aa) over-expression plasmid was capable of expressing the N-end of HTT protein which was an important part for HTT protein shuttling) or SOD1 over-expression plasmid using Fugene HD transfection reagent; after being cultured for 24 hours, starvation treatment was performed for 24 hours, and then the system was replaced with a normal culture system, compound PAO was used for treatment for 4 hours, and the levels of HTT protein and SOD1 protein in the supernatant of cell culture fluid were determined through ELISA. The results showed that the 25 nM, 50 nM, 75 nM, and 100 nM PAO treated groups significantly promoted HTT protein secretion in the PC12 cell line compared to the HTT OE group (FIG. 9A). Meanwhile, in the experiment of SOD1 over-expression, SOD1 secretion in 75 nM and 100 nM PAO treated group had significant significance compared to the HTT OE group (FIG. 9B).

### Example 5. Alleviation of CBE-induced Lysosomal Aggregation by Inhibiting PI4KIIIα

### 5.1 Alleviation of CBE-induced cytotoxicity by PAO promoting lysosomes

Gaucher disease is a common type of lysosome storage disease (LSD). Specifically, because of the activity deficiency of acid β-glucosidase or glucocerebrosidase (GCase) in the body caused by the gene mutation of glucocerebrosidase, the substrate glucocerebroside of glucocerebrosidase is stored in macrophage lysosome of various organs including liver, spleen, bone, lung and even brain, so that the affected tissue and organ have pathological changes, and multiple organs are affected and become progressively aggravated clinically. Conduritol B Epoxide (CBE) is a commonly used inhibitor of β-glucosidase, and CBE is used herein to examine the regulation of Gaucher disease by PAO.

The following concentrations of CBE were added to the cultured SH-SY5Y cell line, respectively: 1.5 µM, 3 µM, 6.25 µM, 12.5 µM, 25 µM, 50 µM and 100 µM, and after 72 hours of treatment cell viability was determined. The results showed that cell death or apoptosis caused by 50 µM and 100 µM of CBE treatment for 72 hours was significantly different compared to the control group (ctrl) (FIG. 10A). 100 µM CBE was selected to treat SH-SY5Y cells for subsequent experiments. After 100 µM CBE treatment for 24 hours, starvation treatment was performed, and after starvation treatment for 24 hours, 100 µM CBE was used for re-incubation and different concentrations of PAO was given for treatment for 24 hours, and MTT assay was performed to determine cell viability rate. n = 5, One-way ANOVA, **p < 0.001, ***p < 0.0001, vs. α-syn-OE. The effect of PAO on cell death or apoptosis caused by 100 µM CBE treatment was assayed by MTT. The results for the experiments showed that the cell viability rate in the 6.25 nM, 12.5 nM, 25 nM, 50 nM, 75 nM and 100 nM PAO-treated groups was significantly higher than that in the 100 µM CBE-treated group (FIG. 10B).

### 5.2 Promotion of lysosomal release and reduction of intracellular accumulation of multiple GlcCer by PAO

Because PAO could induce lysosomal release, the present invention combined PAO and CBE to test whether PAO could affect lysosomal release. Lysosome tracker (LysoTracker) was added to label lysosomes after SH-SY5Y cells were treated with 100 µM CBE for 72 hours. The results showed that the number of LysoTracker-labeled positive cells in the 100 µM CBE-treated group was significantly increased compared to that of the negative control group (FIG. 11), indicating that CBE induced aggregation in lysosome and could be used to simulate Gaucher disease. SH-SY5Y was treated with 100 µM CBE for 72 hours and then LysoTracker was added, and 50 nM or 75 nM PAO was given for treatment for 5 minutes (A) or 10 minutes (B) after 30 minutes, and observation was performed after 4% paraformaldehyde (PFA) fixation. Scale bar= 100 µm. After 5 minutes (FIG. 11A) or 10 minutes (FIG. 11B), Lyso-tracker labeled positive cells were reduced compared to the control group, indicating that PAO may alleviate CBE-induced aggregation in lysosome.

PAO reduced accumulation of glucosylceramide (GlcCer) in CBE-treated cells. The fundamental defects of GD are the lack of activity of glucocerebrosidase that primarily mediates the breakdown of glucocerebroside into glucose and GlcCer, and thus the storage of substrates such as GlcCer occurs in cells from GD patients or CBE treated cells. In order to further explore whether PAO has an effect on GlcCer storage in SH-SY5Y cell model, we measured GlcCer content of different side chains within cells by LC-MS assay. The results showed that the concentration of GlcCer of various side chains in cells treated with 100 µM CBE was much higher than that in the control group (ctrl), and the concentration of GlcCer in the 100 µM CBE and 50 nM PAO co-treated group decreased as compared to the group treated with 100 µM CBE (FIG. 11C).

Compounds A1 and G1 are also specific inhibitors of PI4KIIIα. SH-SY5Y was treated with 100 µM CBE for 72 hours, then Lysosome tracker (Lyso-tracker) was added to label lysosomes; after 30 minutes, 50 nM or 75 nM A1 or G1 was given for treatment for 10 minutes. The results showed that the number of Lyso-tracker labeled positive cells in the 100 µM CBE-treated group was significantly increased compared to the negative control group, and treatment with 50 nM and 75 nM A1 or G1 for 10 minutes resulted in fewer Lyso-tracker labeled positive cells as compared to 100 µM CBE treatment (FIG. 12A, B). Thus, both A1 and G1 could promote exocytosis of lysosomal enzymes, reducing accumulation in lysosome resulting from CBE treatment.

### 5.3 Promotion of lysosomal exocytosis or reduction of lysosomal storage by knockdown of PI4Kα

Previous studies showed that PAO at low concentrations (< 5 µM) acted mainly on phosphatidylinositol 4-kinase PI4KIIIα, and in order to further explore the role of PAO target PI4KIIIα in lysosomal storage diseases and protein misfolding-related diseases, we designed shRNA interfering lentiviral vectors (with green fluorescent protein GFP expression sequence) against the gene sequence PI4Kα encoding PI4KIIIα protein. The results for Western blot assay showed that 48 hours after transfection of SH-SY5Y cells with three shRNA interfering lentiviral vectors against PI4Kα, the interfering sequences sh1, sh2 and sh3 significantly reduced the expression level of PI4KIIIα in the treated group (FIGs. 13A and 13B). The fluorescence intensity of the Lyso-tracker was observed by immunofluorescence 48 hours after the treatment of the shRNA interfering lentiviral vectors. Compared with sh-ctrl, the immunofluorescence intensity of Lyso-tracker in sh-ctrl and 100 µM CBE co-treated group increased significantly. Compared with the sh-ctrl and 100 µM CBE co-treated group, the immunofluorescence intensity of Lyso-tracker in the sh1-PI4Kα and 100 µM CBE co-treated group decreased significantly. The above results indicate that knockdown of PI4Kα can reduce lysosomal storage or promote lysosomal exocytosis.

### Example 6. Promotion or Activation of Autophagy and Activation of Autophagic Flux by Inhibiting PI4KIIIα

Previous studies showed that ALP was blocked during development of lysosomal storage diseases such as GD. After SH-SY5Y cells were treated by CBE for 48 hours, PAO or mTOR inhibitor Rapamycin (RAPA) (as positive control) with different concentrations was added respectively, incubated for 24 hours, and subjected to Western blot assay or immunofluorescence assay, and the effect of compounds such as PAO on autophagy-lysosome pathway was researched by observing common markers to ALP. The markers LC3B and p62 common to ALP were detected, and western blot assay showed: in the SH-SY5Y cell model constructed by CBE, PAO dose-dependently promoted LC3B protein expression and inhibited p62 protein level, indicating that PAO activated the ALP pathway and activated autophagic flux (FIGs. 14A-C), which was similar to the results in the positive control 500 nM RAPA (FIGs. 14A-C). Immunofluorescence results were consistent with western blot assay results (FIG. 14D). In addition, the H+-ATPase inhibitor Bafilomycin A1 (Baf-A1) is a commonly used ALP inhibitor, and the blocking of ALP signaling by Baf-A1 can further verify whether the protection effect of compounds such as PAO on SH-SY5Y cells constructed by CBE is related to ALP. SH-SY5Y cells were treated with CBE for 48 hours, and then PAO at different concentrations or 50 nM Baf-A1 was added according to grouping respectively and incubated for 24 hours, and the cell viability was determined by MTT assay. The experimental results showed that 100 µM CBE significantly inhibited SH-SY5Y cell viability compared to the control group (ctrl). Compared with the 100 µM CBE treated group, cell viability significantly increased in the 100 µM CBE and 25 nM, 50 nM and 75 nM PAO co-treated groups, the 50 nM Baf-A1 treatment decreased the protection effect of PAO in the corresponding groups, and the cell viability of the groups treated with 50 nM Baf-A1 and 100 µM CBE combined with 25 nM, 50 nM and 75 nM PAO, respectively, was not significantly different from the group treated with 100 µM CBE (FIG. 14E), indicating that Baf-A1 blocked the protection effect of PAO on CBE-treated SH-SY5Y cells by inhibiting ALP signaling. The above results demonstrated that PAO activates ALP to activate autophagic flux and exerts protective effect on GD cell model via ALP.

ALP pathway marker LC3B was detected on SH-SY5Y cells treated with shRNA interfering lentiviral vector, and the results showed: compared with the sh-ctrl group, LC3B protein levels were significantly increased after knockdown of PI4Kα (FIG. 15), and knockdown of PI4Kα in CBE-treated SH-SY5Y cells similarly promoted LC3B protein expression, indicating the same result as the PI4Kα inhibitor PAO, and knockdown of PI4Kα also activated the ALP pathway.

### Example 7. Decrease of Intracellular Cholesterol Accumulation in Niemann-Pick Type C (NPC) by Inhibiting PI4KIIIα (FIG. 16)

U18666A, an inhibitor of intracellular cholesterol transport, is often used to construct a cell model of Niemann-Pick Type C (NPC).

### 1 Cell culture and compound treatment

SH-SY5Y cells were cultured in a complete medium of high-glucose DMEM plus 15% FBS in an incubator at 37°C, 5% CO₂. When the cells were 70% confluent, 10 µM U18666A (purchased from Absin Bioscience Inc., Cat. No: abs819512) was added and d5PAO and PAO at different depths were added according to the grouping, and cultured for 24 hours.

### 2 Filipin staining

1) The culture medium in the 24-well plate was discarded, the plate was added with 1 mL PBS buffer and left to stand for 1 minute, the liquid in the 24-well plate was discarded, and the above steps were repeated 2 times;
2) 1 mL of 4% paraformaldehyde was added into each well and fixation was performed at room temperature for 30 minutes;
3) 4% paraformaldehyde in the 24-well plate was discarded, 1 mL PBS buffer was added and the 24-well plate was slightly shaken for 1 minute, the liquid in the 24-well plate was discarded, and the above steps were repeated 2 times;
4) 1 mL of 1.5 mg/mL glycine solution was added to each well, and the plate was incubated at room temperature for 10 minutes;
5) the liquid in the 24-well plate was discarded, and 1 mL of 50 µg/mL Filipin staining solution (purchased from Sigma-Aldrich, Catalog No: SAE0087) was added, and incubation was performed for 1 hour at room temperature in the dark;
6) the liquid in the 24-well plate was discarded, 1 mL PBS buffer was added and the 24-well plate was slightly shaken for 1 minute, the liquid in the 24-well plate was discarded, and the above steps were repeated 2 times;
7) the glass slide was taken, 5 microliters of a mounting medium (containing DAPI) were added dropwise on the center of the glass slide, the cell slide was taken, dried, and covered on the glass slide with the side with the cells facing downwards to ensure that the cell slide was fully contacted with the mounting medium, and the cells were incubated for 30 minutes at room temperature in the dark.
8) Observation was performed by using a laser confocal microscope.

### Experimental results

SH-SY5Y cells were treated with 10 µM U18666A, incubated for 24 hours with PAO at different concentrations added according to the grouping, and observed after Filipin staining. Immunofluorescence staining results indicated that the Filipin staining fluorescence intensity in the 10 µM U18666A-alone treated group was enhanced as compared to the control group (ctrl), suggesting that the 10 µM U18666A treatment resulted in an increase in the amount of cholesterol bound to Filipin, *i.e.*, cholesterol storage. The Filipin staining fluorescence intensity in the 10 µM U18666A and 35 nM, 70 nM PAO co-treated group decreased as compared to the 10 µM U18666A-alone treated group (FIG. 16). The above results indicate that certain concentrations of PAO can inhibit cholesterol storage caused by U18666A.

It will be apparent to those skilled in the art that, although specific embodiments of the present invention have been described herein for purposes of illustration, various modifications may be made without deviating from the spirit and scope of the present invention. Therefore, the detailed description of embodiments and examples of the present invention should not be construed as limiting the scope of the present invention. The present invention is not limited except as by the appended claims. All documents cited herein are incorporated by reference in their entirety.

## Claims

1. Use of a PI4KIIIα specific inhibitor in preparation of preventing or treating intracellular protein misfolding-related diseases.

2. Use of a PI4KIIIα specific inhibitor in preparation of preventing or treating lysosomal storage diseases.

3. The use according to claim 1 or 2, wherein the PI4KIIIα specific inhibitor is an antibody, a small molecule compound, an RNAi molecule or an antisense nucleic acid.

4. The use according to claim 3, wherein the antibody is a monoclonal antibody or a polyclonal antibody.

5. The use according to claim 3, wherein the antibody is a chimeric antibody, a humanized antibody or a fully human antibody.

6. The use according to claim 3, wherein the RNAi molecule is a small interference RNA (siRNA), a short hairpin RNA (shRNA) or a microRNA (miRNA).

7. The use according to claim 3, wherein the RNAi molecule is 18-100 bases in length.

8. The use according to any of claims 3 and 6-7, wherein the RNAi molecule is modified to enhance its stability.

9. The use according to claim 1 or 2, wherein the PI4KIIIα specific inhibitor is a small molecule compound.

10. The use according to claim 9, wherein the small molecule compound is phenylarsine oxide or a derivative thereof, G1 and an analog thereof, and A1 and an analog thereof.

11. The use according to claim 10, wherein the phenylarsine oxide and the derivative thereof have a structure as represented by formula (I) or a pharmaceutically acceptable salt thereof, 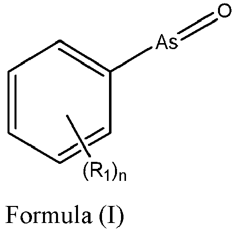 wherein each R₁ is independently selected from (a) H, halogen, nitro, cyano, hydroxyl, amino, carbamoyl, C₁₋₆ alkylsulfuryl, C₁₋₆ alkyl, C₁₋₆ cycloalkyl, C₂₋₆ alkynyl, C₂₋₆ alkenyl, C₁₋₆ alkoxy, C₁₋₆ haloalkyl, C₁₋₆ alkylene-NH₂, C₁₋₆ alkylene-NH-C(O)H, -As(O), -N=NH, N-(C₁₋₆ alkyl)amino, N,N-(C₁₋₆ alkyl)₂ amino, -NH-C(O)H, -NH-S(O)₂H, -C(O)OH, -OC(O)H, -SH, -S(O)₂H, -S(O)₂-NH₂ or heterocyclyl, and optionally substituted with R₂ or R₃, wherein the R₂ and the R₃ are each independently selected from amino, C₁₋₆ alkyl, C₁₋₆ alkoxy, C₁₋₆ haloalkyl, *N*-(C₁₋₆ alkyl) amino, N (6-12 membered aryl)amino, *N*,*N*-(C₁₋₆ alkyl)₂ amino, C₃₋₆ cycloalkyl, 6-12 membered aryl or 3-12 membered heterocyclyl, and optionally substituted with one or more halogen, nitro, cyano, hydroxyl, amino, carbamoyl, -NH-C(O)-Rs, -C(O)OR₄, 6-12 membered aryl, C₁₋₆ alkyl, C₂₋₆ alkynyl, C₂₋₆ alkenyl, C₁₋₆ alkoxy, C₁₋₆ haloalkyl, 3-6 membered heterocyclyl, C₃₋₆ cycloalkyl or Bn-O-, R₄ is C₁₋₆ alkyl, and optionally substituted with one or more halogen, nitro, cyano, hydroxyl, amino, carbamoyl, 6-12 membered aryl, C₁₋₆ alkyl, C₂₋₆ alkynyl, C₂₋₆ alkenyl, C₁₋₆ alkoxy, C₁₋₆ haloalkyl, 3-6 membered heterocyclyl, C₃₋₆ cycloalkyl or Bn-O-, and R₅ is selected from H, C₁₋₆ alkyl, C₂₋₆ alkynyl, C₂₋₆ alkenyl, C₁₋₆ alkoxy or C₁₋₆ haloalkyl, and/or
(b) R₁ on two adjacent carbon atoms forms 5-12 membered cycloalkyl, aryl or heterocyclyl, and is optionally substituted with one or more halogen, nitro, cyano, hydroxyl, amino, carbamoyl, 6-12 membered aryl, C₁₋₆ alkyl, C₂₋₆ alkynyl, C₂₋₆ alkenyl, C₁₋₆ alkoxy, C₁₋₆ haloalkyl, 3-6 membered heterocyclyl, C₃₋₆ cycloalkyl or Bn-O-,
wherein n is an integer from 0 to 5.

12. The use according to claim 11, wherein n is an integer from 0 to 2, and each of the R₁ is independently selected from H, halogen, nitro, cyano, hydroxyl, amino, carbamoyl, C₁₋₆ alkylsulfuryl, C₁₋₆ alkyl, C₁₋₆ cycloalkyl, C₁₋₆ alkoxy, C₁₋₆ haloalkyl, -As(O), *N*-(C₁₋₆ alkyl)amino, *N*,*N*-(C₁₋₆ alkyl)₂ amino, -NH-C(O)H or -NH-S(O)₂H, and optionally substituted with the R₂ or the R₃.

13. The use according to claim 11, wherein n is an integer from 0 to 2, and each of the R₁ is independently selected from H, halogen, nitro, cyano, hydroxyl, amino, C₁₋₆ alkylsulfuryl, C₁₋₆ alkyl, C₁₋₆ cycloalkyl, C₁₋₆ alkoxy, C₁₋₆ haloalkyl, -As(O), -NH-C(O)H or - NH-S(O)₂H, and optionally substituted with the R₂ or the R₃.

14. The use according to claim 11, wherein n is 1 or 2, each of the R₁ is independently selected from H, halogen, amino, C₁₋₆ alkylsulfuryl, C₁₋₆ cycloalkyl, C₁₋₆ alkoxy, C₁₋₆ haloalkyl, -NH-C(O)R₂ or -NH-S(O)₂R₃, wherein the R₂ is C₁₋₆ alkyl and optionally substituted with one 6-12 membered aryl, and the R₃ is 6-12 membered aryl and optionally substituted with one halogen, C₁₋₆ alkoxy or C₁₋₆ haloalkyl.

15. The use according to claim 14, wherein the R₁ is located at ortho- and/or para-position of -As(O).

16. The use according to claim 11, wherein n is 0.

17. The use according to claim 10, wherein the small molecule compound is selected from the group consisting of the following compounds:

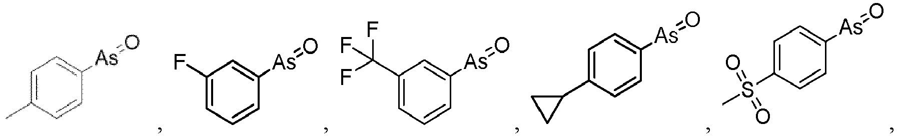

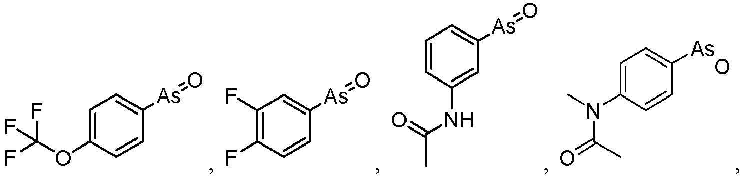

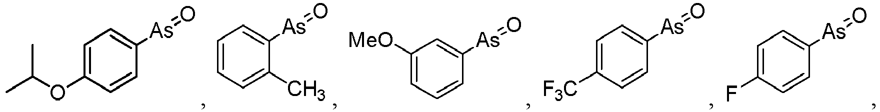

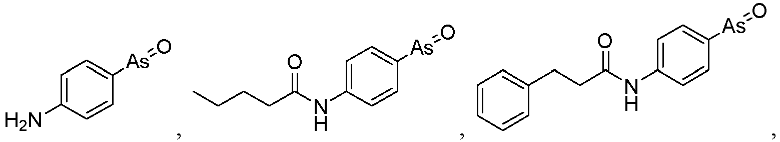

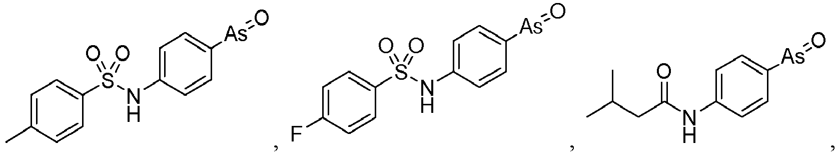

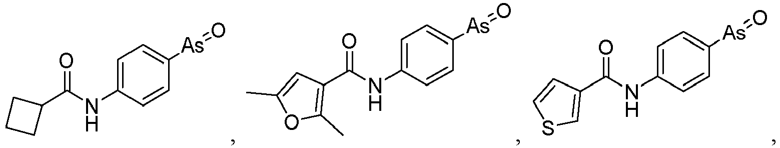

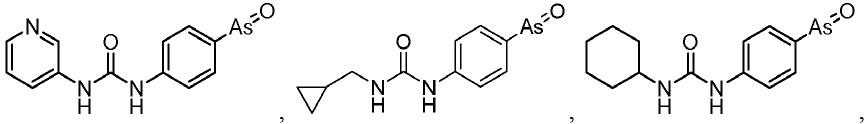

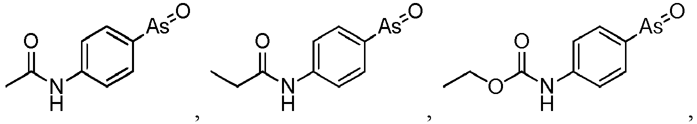

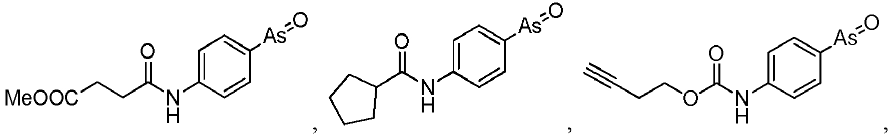

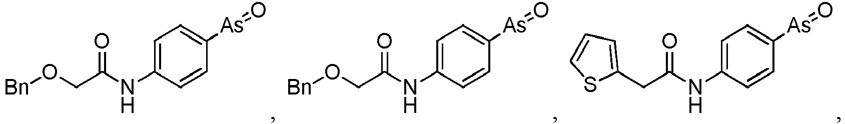

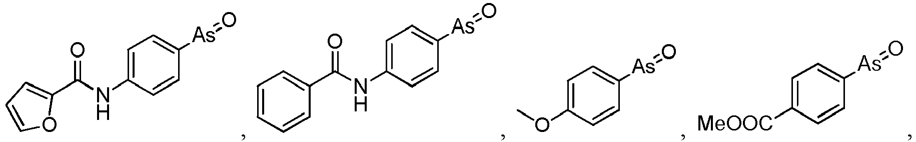

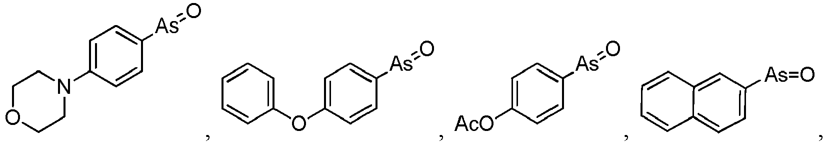

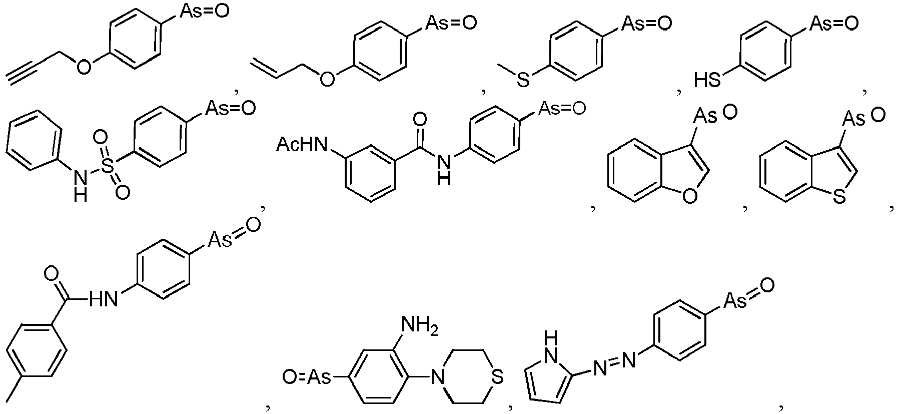

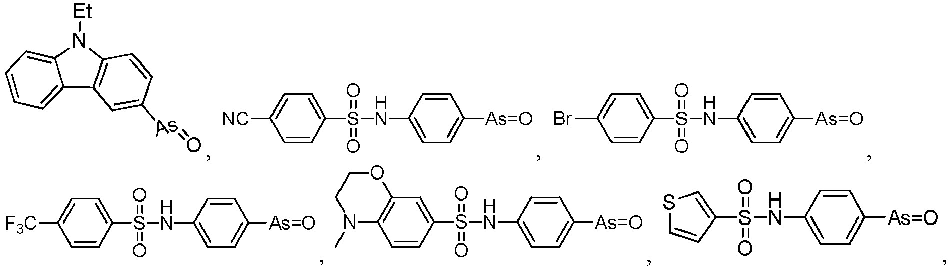

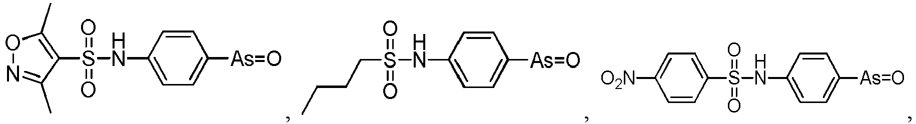

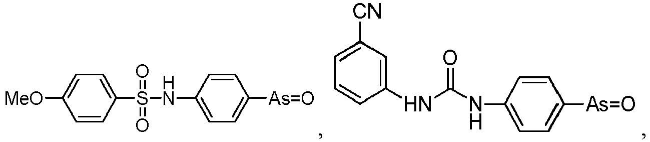

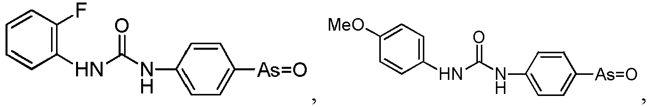

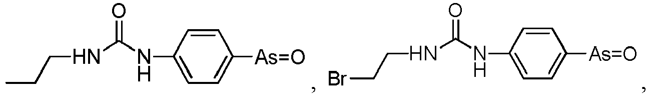

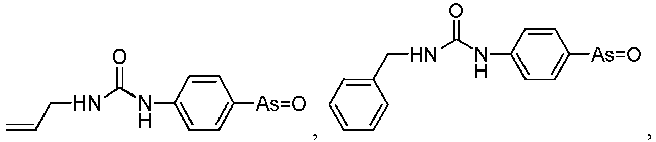

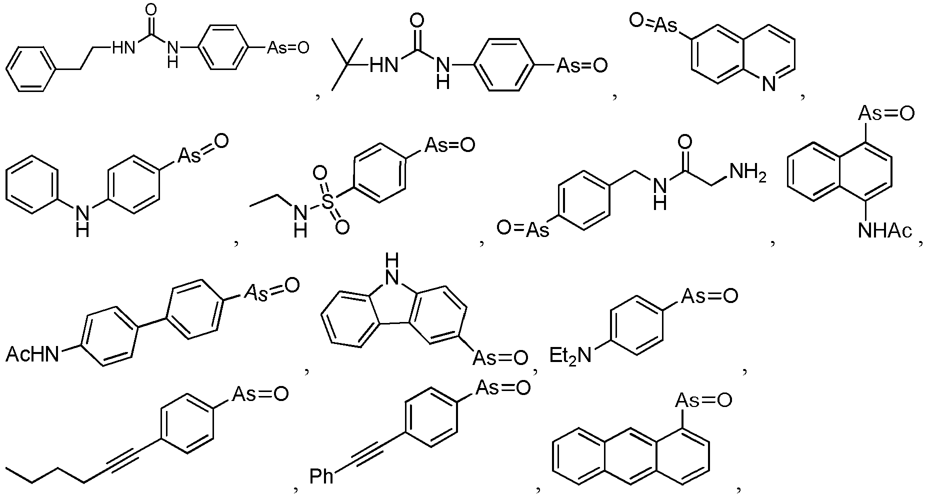

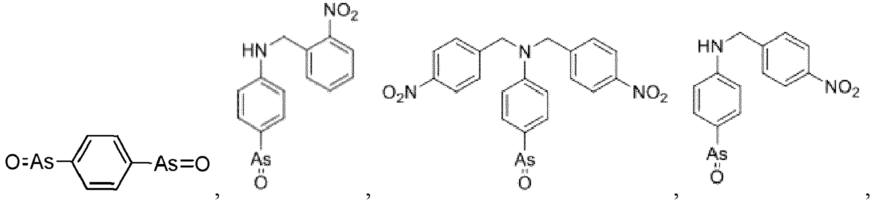

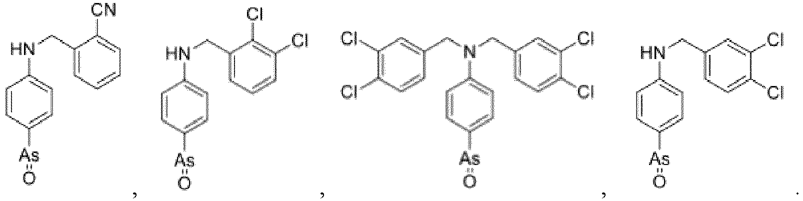

18. The use according to claim 1 or 2, wherein the subject is a human or a mammal.

19. The use according to claim 1, wherein the intracellular protein misfolding-related disease is Parkinson's disease, Lewy body dementia, multiple system atrophy, inclusion body myositis, frontotemporal dementia, Hungtington disease, polyglutamine disease, amyotrophic lateral sclerosis, or Prion disease.

20. The use according to claim 2, wherein the lysosomal storage disease is sphingolipid metabolism disorder, Niemann-Pick type C, mucopolysaccharidosis, glycogen storage disease, glycoprotein storage disease, lipid storage disease, post-translational modification deficiency, integral membrane protein deficiency disorder, neuronal ceroid lipofuscinosis, or lysosome-related organelle disorder.

21. The use according to claim 20, wherein the sphingolipid metabolism disorder is Gaucher disease.

22. The use according to claim 1 or 2, further comprising administering a second agent to a subject in need thereof.

23. The use according to claim 22, wherein the second agent is an agent for treating intracellular protein misfolding-related diseases or an agent for treating lysosomal storage diseases.

24. The use according to claim 23, wherein the PI4KIIIα specific inhibitor is administered prior to, subsequent to or concurrently with the second agent.

25. A method for screening a drug for preventing or treating intracellular protein misfolding-related diseases, comprising contacting a drug candidate with a PI4KIIIα protein or nucleic acid or PI4KIIIα or a PI4KIIIα protein complex, and detecting whether the drug candidate is capable of inhibiting the formation or activity of the PI4KIIIα, or the formation, stability, activity or localization on a cell membrane of the PI4KIIIα protein complex.

26. A method for screening a drug for preventing or treating lysosomal storage diseases, comprising contacting a drug candidate with a PI4KIIIα protein or nucleic acid or PI4KIIIα or a PI4KIIIα protein complex, and detecting whether the drug candidate is capable of inhibiting the formation or activity of the PI4KIIIα, or the formation, stability, activity or localization on a cell membrane of the PI4KIIIα protein complex.

27. The method according to claims 25 and 26, wherein the PI4KIIIα protein complex is a protein complex consisting of PI4KIIIα and Efr3a/Efr3b, TTC7A/TTC7B, FAM126A/FAM126B or homologous proteins thereof.
